# EUROPEAN PATENT APPLICATION

(11) **EP 2 034 027 A1**
(43) Date of publication of application: **11.03.2009**
(21) Application number: 07017299.4
(22) Date of filing: 04.09.2007
(51) Int. Cl.: C12Q 1/68

(54) **Molecular markers to predict response to chemotherapy and EGFR family inhibiton by targeted strategies**

(71) Applicant: Siemens Healthcare Diagnostics GmbH, 65760 Eschborn (DE)
(72) Inventor: Brückl, Wolfgang, Dr., 5730 Mittersill (AT); Wirtz, Ralph, Dr., 50677 Köln (DE)
(74) Representative: Maier, Daniel Oliver

(57) **Abstract**

The present invention relates to a method for predicting a clinical response of a patient suffering from or at risk of developing a neoplastic disease towards a given mode of treatment, said method comprising the steps of:
a) obtaining a biological sample from said patient;
b) determining the expression level of at least one ligand from the Vascular endothelial growth factor (VEGF) family and/or of at least one receptor from the Vascular endothelial growth factor receptor (VEGFR) family in said patient,
c) comparing the pattern of expression levels determined in (b) with one or several reference pattern(s) of expression levels; and
d) predicting therapeutic success for said given mode of treatment in said subject or implementing therapeutic regimen targeting the signalling pathway said ligand is related to in said subject from the outcome of the comparison in step (c). (Fig. 7)

## Description

### Field of the invention

The present invention relates to methods for prediction of the therapeutic success of cancer therapy.

### Background of the invention

Lung cancer is the most common cancer in the world and the main cause of cancer death in Many industrialized countries, as for example the United States. It is classified into small-cell lung cancer (SCLC) and non-small cell lung cancer (NSCLC) with frequencies of about 20% and 80%, respectively¹. The prognosis of lung cancer is poor, although promising new chemotherapeutic agents have been developed.

It has been shown from several studies that the epidermal growth factor receptor (EGFR) plays an improtant role in cancer genesis, particularly in lung cancer. Said receptor, also named ErbB-1, is a cell-surface receptor for members of the epidermal growth factor family (EGF-family) of extracellular protein ligands. The epidermal growth factor receptor is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). It has been reported that mutations affecting EGFR expression or activity often result in cancer.

EGFR is a transmembrane protein receptor which is activated by binding of its specific ligands, including epidermal growth factor and transforming growth factor α (TGFα). Upon activation by its growth factor ligands, EGFR undergoes a transition from an inactive monomeric form to an active homodimer, which then stimuates cell growth, tissue proliferation and cell mitosis, the mechanism of which will be described in the following.
The said EGFR comprises a tyrsoine kinase on its intracellular domain. EGFR dimerization stimulates the activity of said tyrosine kinase. As a result, autophosphorylation of five tyrosine (Y) residues in the C-terminal domain of EGFR occurs. These are Y992, Y1045, Y1068, Y1148 and Y1173. This autophosphorylation elicits downstream activation and signaling by several other proteins that associate with the phosphorylated tyrosines through their own phosphotyrosine-binding SH2 domains. These downstream signaling proteins initiate several signal transduction cascades, principally the MAPK, Akt and JNK pathways, leading to DNA synthesis and cell proliferation².

Such proteins modulate phenotypes such as cell migration, adhesion, and proliferation. The kinase domain of EGFR can also cross-phosphorylate tyrosine residues of other receptors it is aggregated with, and can itself be activated in that manner.

There is some evidence that in some cases preformed inactive dimers may also exist before ligand binding. In addition to forming homodimers after ligand binding, EGFR may pair with another member of the ErbB receptor family, such as ErbB2/Her2/neu, to create an activated heterodimer. Moreover, there is evidence that in some cancerogenic cells the overexpression of EGFR leads to an elevated abundance of said receptor in the cellular membranes, which leads to autonomous dimerization due to high receptor density, without the need for the ligand to elicit said dimerization.

Hence, an overexpression of either native or mutant EGFR due is frequently found in cancerogenic and pre-cancerogenic cells and/or tissues. Said overexpression may be accompanied by mutations of the EGFR gene itself, as well as to gene amplification, polysomy, aneuploidy, genomic instability and the like. Said overexpression leads to a self-activation of cell proliferation in the respective cells and/or tissues due to autonomous dimerization. Overexpression of EGFR does thus trigger a positive feedback mechanism which rapidly enforces tumor growth.

Recently, erlotinib (trade name: Tarceva), a so-called small molecular drug acting as highly selective epithelial growth factor receptor (EGF-R) tyrosine kinase inhibitor, has been demonstrated to be effective in terms of survival in second/third-line chemotherapy in non small cell lung cancer (NSCLC). However, the overall response rate is about 9% ². Independent predictive clinical factors for response to erlotinib include: adenocarcinoma, female sex, Asian origin and never having smoked ^{3,4}. There is evidence that the said medicament is particularly effective in tumors bearing EGFR mutations. Similar effects have been reported for Gefitinib (trade name: iressa).

Additionally, somatic EGFR mutations in the tumour, which are commonly clustered in the tyrosine kinase domain of the receptor (exons 18 to 21) or high polysomy of the EGFR gene may be of positive predictive influence ^{5,6}. However, there are patients described, in whose tumours no EGFR-mutation could be found despite showing responses to erlotinib⁷. Therefore, the inventors of the present invention have assumed that besides changes in EGF-R other genetic phenomenon might exist in tumours sensitive to EGF-R inhibitors.

It is yet difficult to determine those tumor types which are promoted by EGFR overexpression. This is due to the fact that an overexpression rate of 2x, which is often enough to promote the above identified phenomena leading to increased malignancy of the tumor, can not be resolved with standard methods, i.e immunohistochemistry (IHC), fluorescent in situ hybridization (FISH) and/or quantitative PCR.

This means that, in tumor diagnosis, many tumor types which are characterized by enhanced EGFR expression and/or EGFR overexpression and are thus potential targets for EGFR inhibitors might remain undetected with standard methods. Hence, chances to treat these tumors with the most adequate treatments available to date are lost.

As EGFR is a member of the ErbB receptor family, it can be assumed that the above mentioned mechanisms are also applicable to the other ErbB receptors introduced above.

As VEGFA is a member of the VEGF family (Vascular endothelial growth factor) family, and related to the PDGF (Platelet derived growth factor) and FGF (Fibrobast growth factor) family, it can be assumed that the above mentioned mechanisms are also applicable to the other growth factors of said families.

Accordingly, as VEGFR1 is a member of the VEGFR (Vascular endothelial growth factor receptor) family, it can be assumed that the above mentioned mechanisms are also applicable to the other growth factors of said families. including the PDGFR and FGF receptor family.

### Definitions

The term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assesment of the malignancy of a tumor, or to the expected survival rate (DFS, disease free survival) of a patient, if the tumor remains untreated.

The term "response marker" relates to a marker which can be used to predict the clinical response of a patient towards a given treatment.

The term "neoplastic lesion" or "neoplastic disease" or "neoplasia" refers to a cancerous tissue this includes carcinomas, (e.g., carcinoma in situ, invasive carcinoma, metastatic carcinoma) and pre-malignant conditions, neomorphic changes independent of their histological origin (e.g. ductal, lobular, medullary, mixed origin). The term "cancer" as used herein includes carcinomas, (e.g., carcinoma in situ, invasive carcinoma, metastatic carcinoma) and pre-malignant conditions, neomorphic changes independent of their histological origin. The term "cancer" is not limited to any stage, grade, histomorphological feature, invasiveness, agressivity or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies and transformations associated with the lung, ovarian, cervix, esophagus, stomach, pancreas, prostate, head and neck, renal cell, colorectal or breast cancer are included. Particularly types of adenocarcinoma are included, as well as all carcinomas of unknown primary (cup-syndroms). The terms "neoplastic lesion" or "neoplastic disease" or "neoplasia" or "cancer" are not limited to any tissue or cell type they also include primary, secondary or metastatic lesions of cancer patients, and also comprises lymph nodes affected by cancer cells or minimal residual disease cells either locally deposited (e.g. bone marrow, liver, kidney) or freely floating throughout the patients body.

The term "neoplastic cells" refer to abnormal cells that grow by cellular proliferation more rapidly than normal. As such, neoplastic cells of the invention may be cells of a benign neoplasm or may be cells of a malignant neoplasm.

Furthermore, the term "characterizing the state of a neoplastic disease" is related to, but not limited to, measurements and assessment of one or more of the following conditions: Type of tumor, histomorphological appearance, dependence on external signal (e.g. hormones, growth factors), invasiveness, motility, state by TNM (2) or similar, agressivity, malignancy, metastatic potential, and responsiveness to a given therapy.

The terms "biological sample" or "clinical sample", as used herein, refer to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, liquor cerebrospinalis, tear fluid, or cells there from. Biological samples may also include sections of tissues such as frozen or fixed sections taken for histological purposes or microdissected cells or extracellular parts thereof. A biological sample to be analyzed is tissue material from a neoplastic lesion taken by aspiration or punctuation, excision or by any other surgical method leading to biopsy or resected cellular material. Such a biological sample may comprise cells obtained from a patient. The cells may be found in a cell "smear" collected, for example, by a nipple aspiration, ductal lavarge, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

The term "therapy modality", "therapy mode", "regimen" or "chemo regimen" as well as "therapy regimen" refers to a timely sequential or simultaneous administration of anti-tumor, aand/or anti vascular, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of the single agent, timeframe of application and frequency of administration within a defined therapy window. Currently various combinations of various drugs and/or physical methods, and various schedules are under investigation.

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

The term "small molecule", as used herein, is meant to refer to a compound which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The terms "modulated" or "modulation" or "regulated" or "regulation" and "differentially regulated" as used herein refer to both upregulation [i.e., activation or stimulation (e.g., by agonizing or potentiating] and down regulation [i.e., inhibition or suppression (e.g., by antagonizing, decreasing or inhibiting)].

The term "transcriptome" relates to the set of all messenger RNA (mRNA) molecules, or "transcripts", produced in one or a population of cells. The term can be applied to the total set of transcripts in a given organism, or to the specific subset of transcripts present in a particular cell type. Unlike the genome, which is roughly fixed for a given cell line (excluding mutations), the transcriptome can vary with external environmental conditions. Because it includes all *mRNA* transcripts in the cell, the transcriptome reflects the genes that are being actively expressed at any given time, with the exception of mRNA degradation phenomena such as transcriptional attenuation. The discipline of transcriptomics examines the expression level of mRNAs in a given cell population, often using high-throughput techniques based on DNA microarray technology.

The term "expression levels" refers, e.g., to a determined level of gene expression. The term "pattern of expression levels" refers to a determined level of gene expression compared either to a reference gene (e.g. housekeeper or inversely regulated genes) or to a computed average expression value (e.g. in DNA-chip analyses). A pattern is not limited to the comparison of two genes but is more related to multiple comparisons of genes to reference genes or samples. A certain "pattern of expression levels" may also result and be determined by comparison and measurement of several genes disclosed hereafter and display the relative abundance of these transcripts to each other.

Alternatively, a differentially expressed gene disclosed herein may be used in methods for identifying reagents and compounds and uses of these reagents and compounds for the treatment of cancer as well as methods of treatment. The differential regulation of the gene is not limited to a specific cancer cell type or clone, but rather displays the interplay of cancer cells, muscle cells, stromal cells, connective tissue cells, other epithelial cells, endothelial cells of blood vessels as well as cells of the immune system (e.g. lymphocytes, macrophages, killer cells).

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

"Primer pairs" and "probes", within the meaning of the invention, shall have the ordinary meaning of this term which is well known to the person skilled in the art of molecular biology. In a preferred embodiment of the invention "primer pairs" and "probes", shall be understood as being polynucleotide molecules having a sequence identical, complementary, homologous, or homologous to the complement of regions of a target polynucleotide which is to be detected or quantified. In yet another embodiment nucleotide analogues are also comprised for usage as primers and/or probes.

"Individually labeled probes", within the meaning of the invention, shall be understood as being molecular probes comprising a polynucleotide, oligonucleotide or nucleotide analogue and a label, helpful in the detection or quantification of the probe. Preferred labels are fluorescent molecules, luminescent molecules, radioactive molecules, enzymatic molecules and/or quenching molecules.

"Arrayed probes", within the meaning of the invention, shall be understood as being a collection of immobilized probes, preferably in an orderly arrangement. In a preferred embodiment of the invention, the individual "arrayed probes" can be identified by their respective position on the solid support, e.g., on a "chip".

The phrase "tumor response", "therapeutic success", or "response to therapy" refers, in the adjuvant chemotherapeutic setting to the observation of a defined tumor free or recurrence free survival time (e.g. 2 years, 4 years, 5 years, 10 years). This time period of disease free survival may vary among the different tumor entities but is sufficiently longer than the average time period in which most of the recurrences appear. In a neo-adjuvant therapy modality, response may be monitored by measurement of tumor shrinkage and regression due to apoptosis and necrosis of the tumor mass.

The term "recurrence" or " recurrent disease" includes distant metastasis that can appear even many years after the initial diagnosis and therapy of a tumor, or local events such as infiltration of tumor cells into regional lymph nodes, or occurrence of tumor cells at the same site and organ of origin within an appropriate time.

"Prediction of recurrence" or "prediction of therapeutic success" does refer to the methods described in this invention. Wherein a tumor specimen is analyzed for it's gene expression and furthermore classified based on correlation of the expression pattern to known ones from reference samples. This classification may either result in the statement that such given tumor will develop recurrence and therefore is considered as a "non responding" tumor to the given therapy, or may result in a classification as a tumor with a prolonged disease free post therapy time.

"Biological activity" or "bioactivity" or "activity" or "biological function", which are used interchangeably, herein mean an effector or antigenic function that is directly or indirectly exerted by a polypeptide (whether in its native or denatured conformation), or by any fragment thereof *in vivo* or in vitro. Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "marker" or "biomarker" refers to a biological molecule, e.g., a nucleic acid, peptide, protein, hormone, etc., whose presence or concentration can be detected and correlated with a known condition, such as a disease state.

The term "ligand", as used herein, relates to a molecule that is able to bind to and form a complex with a biomolecule to serve a biological purpose. In a narrower sense, it is an effector molecule binding to a site on a target protein, by intermolecular forces such as ionic bonds, hydrogen bonds and Van der Waals forces. The docking (association) is usually reversible (dissociation). Actual irreversible covalent binding between a ligand and its target molecule is rare in biological systems. Ligand binding to receptors often alters the chemical conformation, i.e. the three dimensional shape of the receptor protein. The conformational state of a receptor protein determines the functional state of a receptor. The tendency or strength of binding is called affinity. Ligands include substrates, inhibitors, activators, and neurotransmitters.

The term "agonist", as used herein, relates to a substance that binds to a specific receptor and triggers a response in the cell. It mimics the action of an endogenous ligand that binds to the same receptor.

The term "receptor", as used herein, relates to a protein on the cell membrane or within the cytoplasm or cell nucleus that binds to a specific molecule (a ligand), such as a neurotransmitter, hormone, or other substance, and initiates the cellular response to the ligand. Ligand-induced changes in the behavior of receptor proteins result in physiological changes that constitute the biological actions of the ligands.

The term "signalling pathway" is related to any intra- or intercellular process by which cells converts one kind of signal or stimulus into another, most often involving ordered sequences of biochemical reactions out- and inside the cell, that are carried out by enzymes and linked through homones and growth factors (intercellular), as well as second messengers (intracellular), the latter resulting in what is thought of as a "second messenger pathway". In many signalling pathways, the number of proteins and other molecules participating in these events increases as the process eminates from the initial stimulus, resulting in a "signal cascade" and often results in a relatively small stimulus eliciting a large response.

The term "marker gene," as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and head and neck, colon or breast cancer in particular. A marker gene may also have the characteristics of a target gene.

"Target gene", as used herein, refers to a differentially expressed gene involved in cancer, e.g., head and neck, colon or breast cancer in a manner in which modulation of the level of the target gene expression or of the target gene product activity may act to ameliorate symptoms of malignant neoplasia and lung, ovarian, cervix, esophagus, stomach, pancreas, prostate, head and neck, renal cell, colorectal or breast cancer in particular. A target gene may also have the characteristics of a marker gene.

The term "expression level", as used herein, relates to the process by which a gene's DNA sequence is converted into functional protein (i.e. ligands) and particularly to the amount of said conversion.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected.

These approaches are also known as "array based methods". Yet another hybridization based method is PCR, which is described below. When it comes to the determination of expression levels, hybridization based methods may for example be used to determine the amount of mRNA for a given gene.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR)

The term "determining the protein level", as used herein, refers to methods which allow the quantitative and/or qualitative determination of one or more proteins in a sample. These methods include, among others, protein purification, including ultracentrifugation, precipitation and chromatography, as well as protein analysis and determination, including the use protein microarrays, two-hybrid screening, blotting methods including western blot, one- and two dimensional gelelectrophoresis, isoelectric focusing and the like.

The term "method based on the electrochemical detection of molecules" relates to methods which make use of an electrode system to which molecules, particularly biomolecules like proteins, nucleic acids, antigens, antibodies and the like, bind under creation of a detectable signal. Such methods are for example disclosed in WO0242759, WO0241992 and WO02097413 filed by the applicant of the present invention, the content of which is incorporated by reference herein. These detectors comprise a substrate with a planar surface which is formed, for example, by the crystallographic surface of a silicon chip, and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules. The probe molecules are for example immobilized by a Thiol-Gold-binding. For this purpose, the probe is modified at its 5'-or 3'-end with a thiol group which binds to the electrode comprising a gold surface. These target nucleic acid molecules may carry, for example, an enzyme label, like horseradish peroxidise (HRP) or alkaline phosphatase. After the target molecules have bound to the probes, a substrate is then added (e.g., α-naphthyl phosphate or 3,3'5,5'-tetramethylbenzidine which is converted by said enzyme, particularly in a redox-reaction. The product of said reaction, or a current generated in said reaction due to an exchange of electrons, can then be detected with help of the electrical detector in a site specific manner.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

The term "etiopathology" relates to the course of a disease, that is its duration, its clinical symptoms, and its outcome.

The term "detection of a ligand and/or receptor" as used herein means both the qualitative detection of the presence of the respective gene as well as the quantitative detect detection of of the expression level of the respective gene, e.g. by quantitative reverse transcriptase PCR.

The term "nucleic acid molecule" is intended to indicate any single- or double stranded nucleic acid molecule comprising DNA (cDNA and/or genomic DNA), RNA (preferably mRNA), PNA, LNA and/or Morpholino.

The term "stringent conditions" relates to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5° C. lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion (or other salts) at pH 7.0 to 8.3 and the temperature is at least about 30° C. for short probes (e.g. 10 to 50 nucleotides) and at least about 60° C. for longer probes. Stringent conditions may also be achieved with the addition of destabilizing agents, such as formamide and the like.

The term "fragment of the nucleic acid molecule" is intended to indicate a nucleic acid comprising a subset of a nucleic acid molecule according to one of the claimed sequences. The same is applicable to the term "fraction of the nucleic acid molecule".

The term "variant of the nucleic acid molecule" refers herein to a nucleic acid molecule which is substantially similar in structure and biological activity to a nucleic acid molecule according to one of the claimed sequences.

The term "homologue of the nucleic acid molecule" refers to a nucleic acid molecule the sequence of which has one or more nucleotides added, deleted, substituted or otherwise chemically modified in comparison to a nucleic acid molecule according to one of the claimed sequences, provided always that the homologue retains substantially the same binding properties as the latter.

The term "derivative," as used herein, refers to a nucleic acid molecule that has similar binding characteristics to a target nucleic acid sequence as a nucleic acid molecule according to one of the claimed sequences

### Object of the invention

It is one object of the present invention to provide a method for the determination of tumors which are characterized by elevated expression and/or dependency of receptors belonging to the ErbB family, particularly EGFR.

It is another object of the present invention to provide an in vivo method for the determination of tumor tissue which is characterized by elevated expression and/or overexpression of receptors belonging to the ErbB family, and and/or dependency on elevated EGFR activity.

It is yet another object of the present invention to provide an in vitro and/or in vivo method for the determination whether or not a tumor is likely to be susceptible to a medication related to the signalling pathway of receptors from the ErbB receptor family. In a preferred embodiment the invention refers to therapies targeting ErbB family members itself. In yet another embodiment the invention refers to gene products regulated by ErbB family members and downstream activities thereof, such as family members of the VEGF/VEGFR and/or FGF/FGFR system.

These objects are met with methods and means according to the independent claims of the present invention. The dependent claims are related to preferred embodiments.

### Summary of the invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to the invention, a method is provided for predicting a clinical response of a patient suffering from or at risk of developing a neoplastic disease towards a given mode of treatment. Said method comprises the steps of:
a) obtaining a biological sample from said patient;
b) determining the expression level of at least one ligand from the Vascular endothelial growth factor (VEGF) family and/or of at least one receptor from the Vascular endothelial growth factor receptor (VEGFR) family in said patient,
c) comparing the pattern of expression levels determined in (b) with one or several reference pattern(s) of expression levels; and
d) predicting therapeutic success for said given mode of treatment in said subject or implementing therapeutic regimen targeting the signalling pathway said ligand is related to in said subject from the outcome of the comparison in step (c).

In a preferred embodiment of the present invention, it is provided that the mode of treatment for which prediction is sought is a treatment related to the signalling pathway of receptors from the ErbB receptor family.

In yet another preferred embodiment of the present invention, it is provided that the mode of treatment for which prediction is sought is a treatment related to the signalling pathway of a ligand from the VEGF family and/or of a receptor from the VEGFR family.

The ErbB family of receptors, comprises four closely related receptor tyrosine kinases, namely
- EGFR (ErbB-1) (epidermal growth factor receptor), also known as ErbB-1 or HER1
- HER2 (ErbB-2; Her-2/neu),
- HER3 (ErbB-3), and
- HER4 (ErbB-4).

There is evidence that any of these receptors may be related to cancer genesis, as well as to an enhanced expression of VEGF ligands and/or VEGFR receptors. However, a preferred receptor of the method according to the invention is EGFR, as there is
- a number of medicaments available which relate to the EGFR signalling pathway,
- the interaction between EGFR and VEGF expression has been clearly demonstrated,
- the role of EGFR in tumor genesis is well known, and
- the therapeutic effect of tumor medication directed to the EGFR signalling pathway has been demonstrated.

The VEGF family of growth factor ligands comprises several members which all have in common that they feature a cystine-knot domain, and bind to tyrosine kinase receptors, like those from the ErbB family. The VEGF family comprises, apart from the vascular endothelial growth factors (VEGF), the Placenta growth factor (PlGF), as well as Platelet derived growth factors (PDGF). Particularly, the following growth factors belong to the VEGF family:
- VEGF-A,
- VEGF-B,
- VEGF-C,
- VEGF-D (FIGF),
- PDGF-A,
- PDGF-B,
- PDGF-C, and/or
- PlGF.

A number of other VEGF-related proteins have also been discovered encoded by viruses (VEGF-E) and in the venom of some snakes (VEGF-F).

All of these growth factors are ligands which are related to the ErbB signalling pathway, as their expression level is upregulated upon activation or self activation of a receptor of the ErbB family, particularly of EGFR. The growths factors do thus meet the above identified definition according to which the said ligand is related to the signalling pathway of receptors from the ErbB receptor family.

The VEGFR family of growth factor receptors is most commonly expressed in epithelial tissues, like blood vessels and the like. These growth factor receptors comprise a tyrosin kinase which is activated by binding of a respective ligand. The said family comprises the following receptors:
- VEGFR-1 (FLT-1)
- FLT-3
- VEGFR-3 (FLT-4)
- VEGFR-2 (KDR)
- PDGFR-A
- PDGFR-B
- PDGFR-L

Most of the receptors of the VEGFR family are basically expressed in vascular endothelial tissue, i.e. blood vessels and the like. In this context, it is one particular tumor strategy to increase VEGFA expression and thus to promote vascular growth (angiogenesis), in order to enhance vascularization, and thus to promote its oxygen and nutrient supply.

The above mentioned targets, i.e. receptors and/or ligands the expression level of which is used for predicting therapeutic success for said given mode of treatment according to the present invention are listed in table 3.

However, the inventors have found evidence that in non-vascular neoplastic tissue characterized by a defect in EGFR expression (i.e. overexpression or expression of a dysfuntcional or autoactivatable EGFR), VEGFR receptors are expressed as well. This is quite surprising, because, as stated above, the said neoplastic tissues are often non-vascular. However, both EGFR and VEGFR belong to the gfamily of tyrosin kinase receptors. It can be assumed that this phenomon is a strategy the tumor applies to reduce its own dependency from EGFR, particularly, to reduce its susceptibility towards anti-EGFR treatments. However, this means in turn that, in an early tumor stage, anti-EGFR treatments may also contribute to a suppression of VEGFR in the neoplastic tissue. In later stages however, an additional anti-VEGFR treatment may turn out helpful.

In another preferred embodiment of the present invention, it is provided that the method further comprises the steps of
a) obtaining a biological sample from a patient;
b) determining the pattern of expression levels of at least one gene selected from the Vascular endothelial growth factor (VEGF) or Vascular endothelial growth factor receptor (VEGFR) family in said patient,
c) comparing the pattern of expression levels determined in (b) with one or several reference pattern(s) of expression levels;
wherein upregulated expression of at least one ligand determined in (b) is indicative of a promising prediction as regards therapeutic success for a mode of treatment related to the signalling pathway of receptors from the ErbB receptor family.

In this context, other parameters may as well be used and combined in order to predict the therapeutic success for said given mode of treatment. The parameters may be chosen from the group consisting of
- Gender
- Overall histological state
- ECOG performance status
- LDH serum levels

The ECOG performance status is used by doctors and researchers to assess how a patient's disease is progressing, assess how the disease affects the daily living abilities of the patient, and determine appropriate treatment and prognosis ³³.

In yet another preferred embodiment of the present invention, it is provided that said given mode of treatment (a) acts on recruitment of lymphatic vessels, cell proliferation, cell survival and/or cell motility, and/or , and/or b) comprises administration of a chemotherapeutic agent.

Furthermore, in another preferred embodiment of the present invention it is provided that
a) at least one of the said genes the expression level of which is determined is VEGF-A, VEGF-C, VEGFR-1 or VEGFR-2 and/or
b) the mode of treatment for which prediction is sought is a treatment related to the signalling pathway of EGFR.

Among these ligands and/or receptors, VEGF-A and VEGFR-1 are preferred targets, as the inventors of the present invention have shown that in tumors which are susceptible to EGFR inhibitor treatment, the VEGF-A gene is highly upregulated and thus overexpressed. However, similar phenomena can be postulated for the remaining members of the VEGF ligand family and the VEGFR receptor factor family, preferably VEGFC and VEGFR2.

Furthermore, it is provided in an another preferred embodiment of the present invention that said given mode of treatment comprises chemotherapy, administration of small molecule inhibitors, antibody based regimen, anti-proliferation regimen, pro-apoptotic regimen, pro-differentiation regimen, radiation and/or surgical therapy.

In another embodiment of the present invention, method of selecting a therapy modality for a patient afflicted with a neoplastic disease is provided, said method comprising the steps of:
a) obtaining a biological sample from said patient;
b) predicting from said sample, by the method according to the above, therapeutic success for a plurality of individual modes of treatment; and
c) selecting a mode of treatment which is predicted to be successful in step (b).

In a preferred embodiment, said method comprises the steps of
a) obtaining a sample comprising cancer cells from said patient;
b) separately maintaining aliquots of the sample in the presence of one or more test compositions;
c) comparing expression of a single or plurality of molecules, selected from the ligands and/or receptors listed in table 3 in each of the aliquots; and
d) selecting a test composition which induces a lower level of expression of ligands and/or receptors from table 3 and/or a higher level of expression of ligands and/or receptors from table 3 in the aliquot containing that test composition, relative to the level of expression of each ligand in the aliquots containing the other test compositions.

It is particularly preferred that, in the method according to the invention, the said expression level is determined by
a) a hybridization based method;
b) a PCR based method;
c) determining the protein level,
d) a method based on the electrochemical detection of particular molecules, and/or by
e) an array based method.

Furthermore, in a preferred embodiment of the present invention it is provided that said cancer or neoplastic disease is Small cell lung cancer (SCLC), non small cell lung cancer (NSCLC), and/or transformations associated with the ovary, esophagus, stomach, pancreas, prostate, head and neck, renal cell, colon or breast cancer ovarian cancer, cervix, stomach, pancreas, prostate, head and neck, renal cell, colon and/or breast.

In yet another preferred embodiment of the present invention it is provided that that the expression level of at least one of the said ligands is determined with rtPCR (reverse transcriptase polymerase chain reaction) of the ligand related mRNA.

In this preferred embodiment, the mRNA related to VEGF, or to PDGF, respectively, is determined, namely with a reverse transcriptase polymerase chain reaction approach.

The inventors of the present invention have surprisingly found out that the determination of VEGF-A mRNA levels is very informative for the prediction of the clinical response of a patient suffering from or at risk of developing a neoplastic disease owards a medicament related to the signalling pathway of receptors from the ErbB.

In this context, the interaction between the ErbB-receptor family and the VEGF ligand family was studied by correlation analysis. Particular attention was devoted to the EGFR receptor, the VEGFR1 and VEGFR2 and to the VEGF-A ligand.

Surprisingly, it turned out that not the expression of EGFR itself, nor the detection of amplification or mutation, was indicative for a positive prediction, but the expression of the VEGFA ligand and VEGFR1.

One of the major effects of EGFR autophosphorylation seems to be the upregulation of the expression of the Vascular Endothelial Growth Factor (VEGF), which, when being secreted, stimulates, among others, cell proliferation, particulaly angiogenesis.

This is due to the fact that an EGFR overexpression rate of 2x, which is often enough to promote the above identified phenomena leading to increased malignancy of the tumor, can not be resolved with standard methods, i.e immunohistochemistry (IHC), fluorescent in situ hybridization (FISH) and/or quantitative PCR.

In contrast thereto, EGFR-induced VEGF-A overexpression may by higher by orders of magnitude in the said tissues. This means that, while the defect in EGFR expression, which may represent a cause for tumor genesis, can remain undetected under certain circumstances, said defect may lead to a high overexpression of VEGF-A, which may be detected e.g. by quantitative rtPCR. Persistent overexpression of VEGFA by hyperactivated EGFR in turn induces endothelial cell proliferation, migration and formation of new vessels, a biological phenomenen called angiogenesis. Moreover, overexpression of VEGFRs by the tumor cells itself establishes a positive feedback loop driving anti-apoptotic and proliferation activities. Thereby increasing the dependency of tumor cells towards EGFR family signalling. This in turn enables to hit these advanced tumor cells by disrupting this tumor cell specific bypass mechanism.

The inventors have for the first time demonstrated that, if a given rate of VEGF-A and/or VEGFR1 overexpression is detected in a tumor, one may then conclude that the cause for said overexpression is a defect in EGFR expression, which may successfully be treated with drugs related to the EGFR signalling pathway.

Moreover, said overexpression may be taken as a response marker for anti proliferative chemotherapy, as indicated above. VEGF family members, particularly VEGF-A, are known to promote cell growth, in particular tissue proliferation. Potential targets of VEGF-A are, for example, the tumor itself and surrounding blood vessels. This means that the defect in EGFR expression may lead, via excretion of VEGF-A, to enhanced tumor growth as well as to a promotion of tumor vascularization, by promoting the proliferation of surrounding blood vessels (angiogenesis).

The determination of the expression of VEGF-ligands (particularly VEGF-A) may thus be informative both with regard to anti-angiogenic and anti-proliferative therapies as well as with regard to anti-ErbB-therapies (particularly anti-EGFR).

In this context, it seems much more promising to provide anti-ErbB/anti-EGFR treatments, if so indicated, than anti-PDGF/anti-VEGF treatments (for example the anti-VEGF antibody Bevacizumab), because, in the signalling pathway, ErbB/EGFR is located upstream of PDGF/VEGF. This means that, if ErbB/EGFR is inhibited, a number of processes located downstream in the signalling pathway may be suppressed, among which VEGF-induced processes are only one example.

VEGF-related processes may be considered as one symptom out of a range of several symptoms related to tumor genesis, whereas defects in EGFR expression may be considered as the underlying cause for these symptoms, the treatment of which seems thus to be much more promising than the treatment of just one of said symptoms. The inventors of the present invention have for the first time shown these interrelationship.

In addition, it can be concluded that combined anti-ErbB/anti-EGFR treatments and anti-PDGF/anti-VEGF treatments may be most promising in this situation.

In another preferred embodiment of the present invention, it is provided that the expression level of at least one of the said ligands of is determined in formalin and/or paraffin fixed tissue samples.

In yet another preferred embodiment of the present invention, it is provided that the expression level of at least one of the said ligands or receptors is determined in serum, plasma or whole blood samples.

Routinely, in tumor diagnosis tissue samples are taken as biopsies form a patient and undergo diagnostic procedures. For this purpose, the samples are fixed in formaline and/or parrafine and are then examined with immunohistochemistry methods. The formaline treatment leads to the inactivation of enzymes, as for example the ubiquitous RNA-digesting enzymes (RNAses). For this reason, the mRNA status of the tissue (the so called transcriptome), remains undigested.

However, the formaline treatment leads to partial depolymerization of the individual mRNA molecules. For this reason, the current doctrine is that formaline fixed tissue samples can not be used for the analysis of the transcriptome of said tissue.

For this reason, it is provided in a preferred embodiment of the present invention that after lysis, the samples are treated with silica-coated magnetic particles and a chaotropic salt, in order to purify the nucleic acids contained in said sample for further determination.

Collaborators of the inventors of the present invention have developed an approach which however allows successful purification of mRNA out of tissue samples fixed in such manner, and which is disclosed, among others, in WO03058649, WO2006136314A1 and DE10201084A1, the content of which is incorporated herein by reference.

Said method comprises the use of magnetic particles coated with silica (SiO₂). The silica layer is closed and tight and is characterized by having an extremely small thickness on the scale of a few nanometers. These particles are produced by an improved method that leads to a product having a closed silica layer and thus entail a highly improved purity. The said method prevents an uncontrolled formation of aggregates and clusters of silicates on the magnetite surface whereby positively influencing the additional cited properties and biological applications. The said magnetic particles exhibit an optimized magnetization and suspension behavior as well as a very advantageous run-off behavior from plastic surfaces. These highly pure magnetic particles coated with silicon dioxide are used for isolating nucleic acids, including DNA and RNA, from cell and tissue samples, the separating out from a sample matrix ensuing by means of magnetic fields.

These particles are particularly well-suited for the automatic purification of nucleic acids, mostly from biological body samples for the purpose of detecting them with different amplification methods.

The selective binding of these nucleic acids to the surface of said particles is due to the affinity of negatively charged nucleic acids to silica containing media in the presence of chaotropic salts like guanidinisothiocyanate. Said binding properties are known as the so called "boom principle". They are described in the European patent EP819696.

The said approach is particularly useful for the purification of mRNA out of formaline and/or paraffine fixed tissue samples. In contrast to most other approaches, which leave very small fragments behind that are not suitable for later determination by PCR and/or hybridization technologies, the said approach creates mRNA fragments which are large enough to allow specific primer hybridzation and/or specific probe hybridization. A minimal size of at least 100 bp, more preferably 200 base pairs is needed for specific and robust detection of target gene expression. Moreover it is also necessary to not have too many inter-sample variations with regard to the size of the RNA fragments to guarantee comparability of gene expression results. Other issues of perturbance of expression data by sample preparation problems relate to the contamination level with DNA, which is lower compared to other bead based technologies. This of particular importance, as the inventors have observed, that DNAse treatment is not efficient in approximately 10% of FFPE samples generated by standard procedures and stored at room temperature for some years before cutting and RNA extraction.

The said approach thus allows a highly specific determination of candidate gene expression levels with one of the above introduced methods, particularly with hybridization based methods, PCR based methods and/or array based methods, even in formaline and/or paraffine fixed tissue samples, and is thus extremely beneficial in the context of the present invention, as it allows the use of tissue samples fixid with formaline and/or paraffine, which are available in tissue banks and connected to clinical databases of sufficient follow-up to allow retrospective analysis.

In another preferred embodiment said treatment related to the signalling pathway of receptors from the ErbB receptor family comprises the administration of an agonist and/or antagonist of the ErbB receptor domain.

In yet another preferred embodiment said treatment related to the signalling pathway of receptors from the ErbB receptor family comprises the administration of nucleotides, ribozomes, aptamers and /or nucleotide analogues capable of affecting the expression of ErbB receptor, VEGF receptor and /or VEGFA ligand expression. Preferably these substances act via downregulation of mRNA levels of said candidate genes.

However, also induction or elevation of gene expression by introduction of receptor gene expression (e.g. ERBB-4 and/or VEGFD expression) can be advantageous to counteract the tumorpromoting activity (e.g. of EGFR, Her-2/neu and/or VEGFC).

By way of illustration and not by way of restriction said agonists may be selected from the group consisting of Cetuximab (tradename Erbitux^{®}, target receptor is EGFR), Matuzumab (EMD7200, target receptor is EGFR), Trastuzumab (tradename Herceptin^{®}, target receptor is HER2/neu), Pertuzumab (target receptor is HER2/neu), Bevacizumab (tradename Avastin^{®}, target ligand is VEGFA), 2C3 (target ligand is VEGFA), VEGF-trap (AVE-0005, target ligands are VEGFA and PIGF), IMC-1121B (target receptor is VEGFR2), CDP-791 (target receptor is VEGFR2) .

In yet another preferred embodiment said treatment related to the signalling pathway of receptors from the ErbB receptor family comprises the administration of a tyrosin kinase inhibitor.

By way of illustration and not by way of restriction said inhibitors may be seleccted from the group consisting of Gefitinib (tradename Iressa^{®}, ZD-1839, target receptor is EGFR), Erlotinib (tradename Tarceva^{®}, OSI-774, target receptor is EGFR), EKB-569 (target receptor is EGFR), PKI-166 (target receptor is EGFR), ), PKI-166 (target receptor is EGFR), Lapatinib (tradename tycerb^{®}, target receptor is EGFR and Her-2/neu), GW572016 (target receptors are EGFR and Her-2/neu), AEE-788 (target receptors are EGFR, Her-2/neu and VEGFR-2), CI-1033 (target receptors are EGFR, Her-2/neu and Her4), AZD6474 (target receptors are EGFR and VEGFR-2).

However, other treatments related to the ErbB receptor family signalling pathway which fall under the scope of the present invention comprise the administration of Sorafenib (tradename Nexavar^{®}, BAY 43-9005, target receptors are VEGFR-2, VEGFR-3, c-KIT, PDGFR-B, RET and Raf-Kinase), BAY 57-9352 (target receptor is VEGFR-2), Sunitinib (tradename Sutent^{®}, target receptors are VEGFR-1, VEGFR-2 and PDGFR), AG13925 (target receptors are VEGFR-1 and VEGFR-2), AG013736 (target receptors are VEGFR-1 and VEGFR-2), AZD2171 (target receptors are VEGFR-1 and VEGFR-2), ZD6474 (target receptors are VEGFR-1, VEGFR-2 and VEGFR-3), Vandetenib (ZD 7646), Vatalanib PTK-787/ZK-222584 (target receptors are VEGFR-1 and VEGFR-2), CEP-7055 (target receptors are VEGFR-1, VEGFR-2 and VEGFR-3), CP-547 (target receptors are VEGFR-1 and VEGFR-2), CP-632 (target receptors are VEGFR-1 and VEGFR-2), GW786024 (target receptors are VEGFR-1, VEGFR-2 and VEGFR-3), AMG706 (target receptors are VEGFR-1, VEGFR-2 and VEGFR-3), Imatinib mesylate (tradename Glivec^{®}/Gleevec^{®}, target receptors are bcr-abl and c-KIT), BMS-214662 (target enzyme is Ras farnesyl transferase), CCI-779 (target enzyme is mTOR), RAD0001 (tradename everolismus^{®}, target enzyme is mTOR), CI-1040 (target enzyme is MEK), SU6668 (target receptors are VEGFR-2, PDGFR-B and FGFR-1),, AZD6126, CP547632 (target receptors are VEGFRs), CP868596 GW786034 (target receptors are PDGFRs), ABT-869 (target receptors are VEGFRs and PDGFRs), AEE788 (target receptors are VEGFRs and PDGFRs), _{AZD0530} (target enzymes are src and abl), and CEP7055.

In yet another preferred embodiment said treatment related to the signalling pathway of receptors from the ErbB receptor family comprises the administration of chemotherapeutics.

Said chemotherapeutics may be selected from the group consisting of Cyclophosphamid (Endoxan^{®}, Cyclostin^{®}). Adriamycin (Doxorubicin) (Adriblastin^{®}), BCNU (Carmustin) (Carmubris^{®}), Busulfan (Myleran^{®}),Bleomycin (Bleomycin^{®}), Carboplatin (Carboplat^{®}), Chlorambucil (Leukeran^{®}), CisPlatin (Cisplatin^{®}), Platinex (Platiblastin^{®}), Dacarbazin (DTIC^{®};Detimedac^{®}), Docetaxel (Taxotere^{®}), Epirubicin (Farmorubicin^{®}), Etoposid (Vepesid^{®}), 5-Fluorouracil (Fluroblastin^{®}, Fluorouracil^{®}), Gemcitabin (Gemzar^{®}), Ifosfamid (Holoxan^{®}), Interferon alpha (Roferon^{®}), Irinotecan (CPT 11, Campto^{®}), Melphalan (Alkeran^{®}),Methotrexat (Methotrexat^{®}, Farmitrexat^{®}), Mitomycin C (Mitomycin^{®}), Mitoxantron (Novantron^{®}), Oxaliplatin (Eloxatine^{®}), Paclitaxel (Taxol^{®}), Prednimustin (Sterecyt^{®}), Procarbazin (Natulan^{®}), Pemetrexed (Alimta^{®}), Ralitrexed (Tomudex^{®}), Topotecan (Hycantin^{®}), Trofosfamid (Ixoten^{®}), Vinblastin (Velbe^{®}), Vincristin (Vincristin^{®}), Vindesin (Eldisine^{®}), Vinorelbin (Navelbine^{®}).

Furthermore, a kit useful for carrying out one of the said methods, comprising at least
a) a primer pair and/or a probe each having a sequence sufficiently complementary to a gene encoding for a ligand from the VEGF family and/or a receptor from the VEGFR family and/or
b) at least an antibody directed against a ligand from the VEGF family and/or a receptor from the VEGFR family and/or
is provided.

Furthermore, according to the invention the use of
a) an antibody directed against a ligand from the VEGF family and/or a receptor from the VEGFR family,
b) an antisense nucleic acid or a ribozyme inhibiting the expression of a Gene encoding for a ligand from the VEGF family and/or for a receptor from the VEGFR family, or
c) an inactive version of a ligand from the VEGF family and/or of a receptor from the VEGFR family,
as an antagonist for the preparation of a pharmaceutical composition for the treatment of a cancer associated with the signalling pathway of receptors from the ErbB receptor family.

In this context, a primer pair and/or a probe complementary to the gene encoding for VEGF-A and/or VEGFR1 and/or an antibody directed against VEGF-A and/or VEGFR1 are particularly preferred.

In yet another embodiment of the invention a method for correlating the clinical outcome of a patient suffering from or at risk of developing a neoplastic with the presence or non-presence of a defect in ErbB receptor expression, said method comprising the steps of:
a) obtaining a fixed biological sample from said patient;
b) determining the expression level of at least one ligand from the VEGF family and/or one receptor from the VEGFR family in said patient according to any of the above methods, and
c) correlating the pattern of expression levels determined in (b) with said patient's data, said data being selected from the group consisting of etiopathology data, clinical symptoms, anamnesis data and/or data concerning the therapeutic regimen.

The said method is particularly beneficial for epidemiological studies. These studies profit from the fact that large tissue databases exist comprising paraffin and/or formalin fixed tissue samples together with an extensive documentation of the patient's history, including etiopathology data, clinical symptoms, anamnesis data and/or data concerning the therapeutic regimen.

The said methods allows for large scale studies which comprise the correlation of the clinical outcome of a patient suffering from or at risk of developing a neoplastic disease with the presence or non-presence of a defect in ErbB receptor expression. As mentioned above, a preferred ErbB receptor is EGFR, and a preferred VEGF ligand is VEGF-A and VEGF-C, and a preferred VEGFR is VEGFR-1 and VEGFR-2. In order to successfully adopt this approach, the above introduced method for mRNA purification comprising silica coated magnetic beads and chaotropic salts is quite helpful.

Further more, the present invention provides a nucleic acid molecule, selected from the group consisting of
a) the nucleic acid molecule presented as SEQ ID NO: 1 - 81
b) a nucleic acid molecule having a length of 4 - 80 nucleotides, preferably 18 - 30 nucleotides, the sequence of which corresponds to the sequence of a single stranded fragment of a gene encoding for a ligand and/or receptor selected from the group consisting of VEGFA, VEGFB, VEGFC, FIGF / VEGFD, EGFR / HER-1, ERBB2 / Her-2/neu / HER-2, ERBB3 / HER-3, ERBB4 / HER-4, FLT-1 / VEGFR-1, FLT-3, FLT-4 / VEGFR-3, KDR / VEGFR-2, PDGFA, PDGFB, PDGFC, PDGFRA, PDGFRA, PDGFRB and/or PDGFRL,
c) a nucleic acid molecule that is a fraction, variant, homologue, derivative, or fragment of the nucleic acid molecule presented as SEQ ID NO: 1 - 81
d) a nucleic acid molecule that is capable of hybridizing to any of the nucleic acid molecules of a) - c) under stringent conditions
e) a nucleic acid molecule that is capable of hybridizing to the complement of any of the nucleic acid molecules of a) - d) under stringent conditions
f) a nucleic acid molecule that is capable of hybridizing to the complement of a nucleic acid molecule of e
g) a nucleic acid molecule having a sequence identity of at least 95 % with any of the nucleic acid molecules of a) - f)
h) a nucleic acid molecule having a sequence identity of at least 70 % with any of the nucleic acid molecules of a) - f)
i) a complement of any of the nucleic acid molecules of a) - h), or
i) a nucleic acid molecule that comprises any nucleic acid molecule of a) - i).

See table 6 for a sequence listing. These nucleic acids are being used either as primers for a polymerase chain reaction protocol, or as detectable probes for monitoring the said process.

Furthermore it is provided that the said nucleic acid is selected from the group consisting of RNA, DNA, PNA LNA and/or Morpholino. The nucleic acid may, in a preferred embodiment, be labelled with at least one detectable marker. This feature is applicable particularly for those nucleic acids which serve as detectable probes for monitoring the polymerase chain reaction process

Such detectable markers may for example comprise at least one label selected from the group consisting of fluorescent molecules, luminescent molecules, radioactive molecules, enzymatic molecules and/or quenching molecules.

In a particularly preferred embodiment, the said detectable probes are labeled with a fluorescent marker at one end and a quencher of fluorescence at the opposite end of the probe. The close proximity of the reporter to the quencher prevents detection of its fluorescence; breakdown of the probe by the 5' to 3' exonuclease activity of the taq polymerase breaks the reporter-quencher proximity and thus allows unquenched emission of fluorescence, which can be detected. An increase in the product targeted by the reporter probe at each PCR cycle therefore causes a proportional increase in fluorescence due to the breakdown of the probe and release of the reporter.

In another preferred embodiment of the present invention, a kit of primers and/or detection probes is provided, comprising at least one of the nucleic acids according to the above enumeration and/or their fractions, variants, homologues, derivatives, fragments, complements, hybridizing counterparts, or molecules sharing a sequence identity of at least 70%, preferably 95 %.

Said kit may, in another preferred embodiment, comprise at least one of the nucleic acid molecules presented as SEQ ID NO: 1 - 81, and/or their fractions, variants, homologues, derivatives, fragments, complements, hybridizing counterparts, or molecules sharing a sequence identity of at least 70%, preferably 95 %, and may be used for the detection of a ligand from the VEGF family and/or a receptor from the VEGFR family.

In yet in another preferred embodiment, said kit may comprise at least one of the nucleic acid molecules presented as SEQ ID NO: 1 - 21, and may be used for the detection of different isoforms of VEGFA.

Such kit is especially useful, as it allows to further differentiate the different types of VEGFA which are overexpressed, in order to obtain a more precise diagnosis. To be able to do so, is vital to use sequences (reverse and forward primers and, optionally, detectable probe) which bind to subsequences of the said isoforms with high specifity, and which do thus allow a differentiation of the said isoforms. Surprisingly, the kit presented here meets these demands and has thus exceeded the expectations.

Furthermore, the use of a nucleic acid according as recited above, or of a kit as recited above for the prediction of a clinical response of a patient suffering from or at risk of developing a neoplastic disease towards a given mode of treatment is provided.

### Brief description of the examples and drawings

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.

### Example 1

A 37 year old Caucasian woman presenting with a 6 months history of coughing with haemoptysis a weight loss of 5 kg in 6 months was admitted to hospital on 3^{rd} of October 2005. The patient has never smoked. Apart from an allergy against cat hair, she didn't suffer from any diseases and she didn't take any regular medication.

An x-ray of the lung was performed showing a round structure in the left lower lobe with increased lymph nodes at the left hilus. In the flexible bronchoscopy the left segmental ostium 9 was closed. Snap biopsies of this region revealed a low grade large cell NSCLC. Additionally, an ultrasound of the abdomen was performed detecting multiple solid structures in both liver lobes, with a maximum diameter of 22 mm, suspicious of liver metastases. The pathological examination of a liver biopsy confirmed liver metastasis of the primary NSCLC. Furthermore, a centigram of the bones suspected bone metastases at the left iliac spine, confirmed by an MRT of the pelvis. The investigations were completed by CT scans of the thorax and abdomen, which resulted in an UICC IV tumour stage.

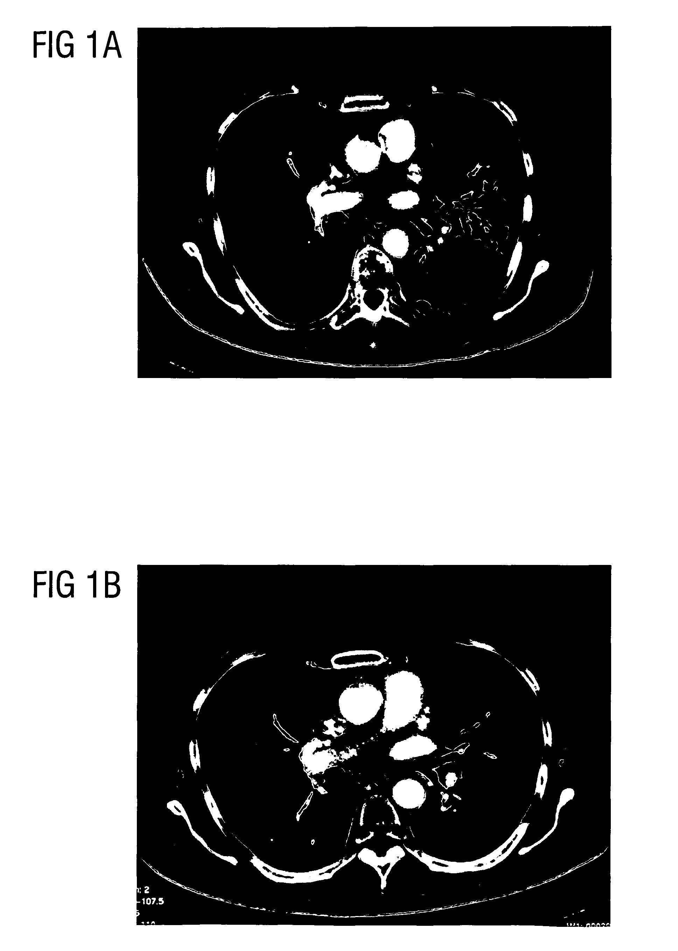

The patient was given palliative first line chemotherapy, consisting of carboplatin 300 mg/m² and docetaxel 75 mg/m² day 1. Chemotherapy was repeated at day 22 and after another 21 days a staging was performed. Progressive disease was evident with an increased primary tumour (Fig. 1A) with increased and additional metastases in the liver (Fig. 1B). Furthermore, a metastasis in the left adrenal gland and diffuse metastatic pattern in the bones were diagnosed. Additionally, brain metastases were present on cranial CT. A fractional cranial irradiation with a total dose of 50 Gy was applied, beginning on 8^{th} of December. As the performance status was still acceptable (EGOG-PS 2) a palliative second line oral therapy with the EGF-R tyrosine kinase inhibitor erlotinib (150mg/d) was started on 14^{th} of December 2005.

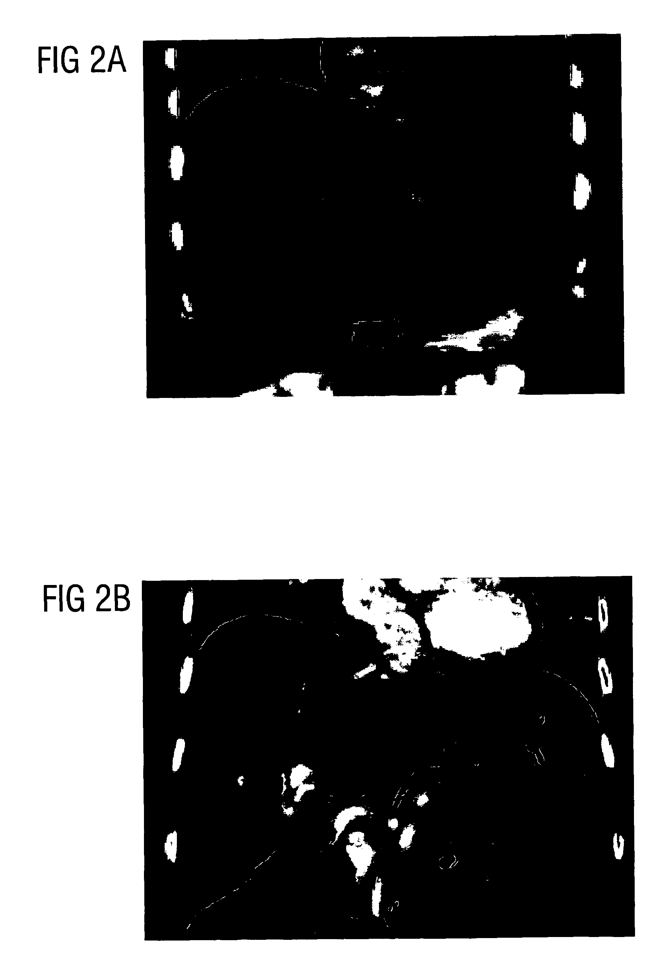

On 10^{th} of February 2006 the patient (2 months erlotinib) had a further tumour staging, showing a partial remission of both the primary tumour and at all metastatic suites. Brain metastasis was no longer evident. Furthermore, the bone metastases had been changed into osteosynthesis. As the patient reported about further hemoptysis, the left chest was fractional irradiated with a total dose of 30 Gy. On 11^{th} of April 2006 (4 months erlotinib) staging confirmed the partial response and showed a further reduction in size of the primary tumour (Fig. 2A) and the liver metastases (Fig. 2B).

The patient was admitted by the emergency unit on 30^{th} of May 2006 with progredient haemoptysis. Progressive disease in the liver and recurrence of metastases in the bones was evident on CT. About ten months after the initial diagnosis of metastatic NSCLC, the patient died with progredient kachexia and dyspnoea due to recurrent pulmonal bleeding on 1^{st} of July 2006. The husband refused an autopsy of the patient. Therefore, it was not possible to take any further tumour tissue and there was no information about the total capacity of the tumour and the metastases.

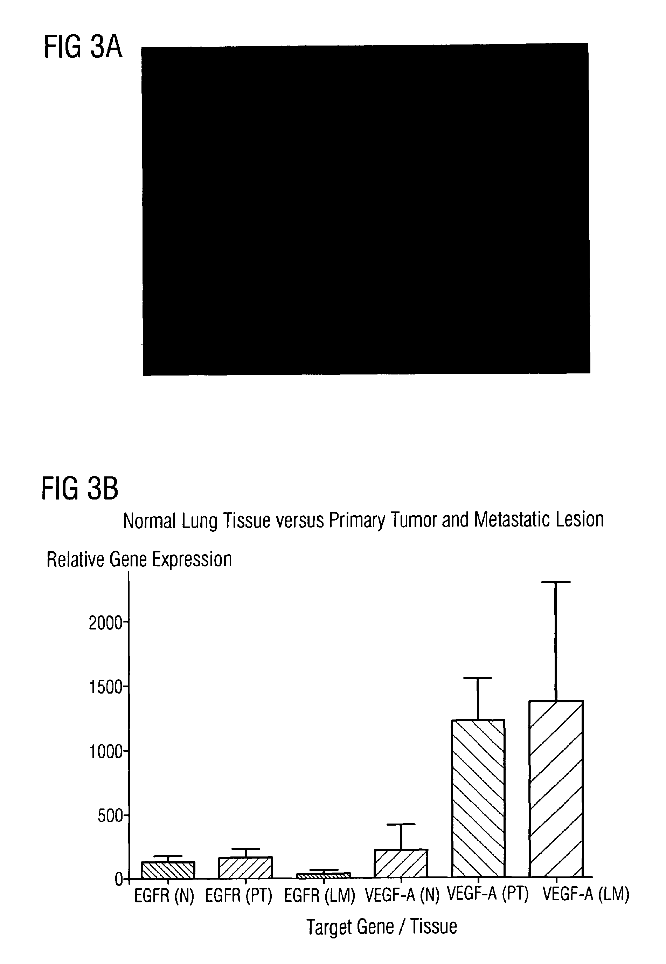

Formalin fixed paraffin embedded tissue from biopsies of primary tumour and the liver metastasis taken during initial diagnosis, was laser microdissected. DNA and RNA were isolated separately from the tumour cells and normal epithelial tissue, respectively. Additionally, DNA from normal lymphocytes of the patient was available. Exons 18 to 21 of the EGF-R gene were bi-directionally sequenced and no somatic mutation could be. detected neither in the primary nor in the liver metastasis (data not shown) ¹⁶. Chromogenic in situ Hybridisation (CISH) analysis showed only a low polysomy of the EGF-R according to the categories defined by Cappuzzo et al.⁸ (Fig. 3A). Additionally, using tumour RNA, gene expression analysis on multiple genes was performed by RT-PCR. While there was an evident overexpression of EGF-R in the primary tumor (lower were detected in the liver metastasis), vascular endothelial growth factor (VEGF-A) gene was greatly upregulated in the primary and in the liver metastases in contrast to normal lung epithelial cells (Fig. 3B). The level of expression of VEGF-B and VEGF-C did not differ significantly between normal and tumour tissue(s).

Angiogenesis has been demonstrated to be an important event in the process of tumour growth and metastatic dissemination. In this context VEGF is a well established key regulator of new blood vessel formation. To our knowledge, there is no report published so far about a clinical response of a VEGF overexpressing NSCLC to an EGF-R therapy. We have found that VEGFA is of predictive relevance in this context. As there was neither a somatic EGF-R mutation found in exons 18 to 21 of the gene in the tumour nor high polysomy of the EGF-R gene, which both have been associated with response ^{4,6}.Also EGFR mRNA overexpression could not be determined in the metastatic leasion of the liver metastasis, while the dramatic overexpression of VEGFA did persist. Activation of the VEGF receptor pathway leads to a number of signal processes promoting proliferation, migration, invasion of endothelial cells and their organization into functional tumour structures, mobilization of endothelial progenitor cells (EPCs) from the bone marrow into peripheral circulation, surviving of pre-existing vessels and an increase in vessel permeability ⁹. In human cancer, overexpression of VEGF has been associated with tumour progression and poor outcome in a variety of solid tumours, e.g. in gastric, pancreatic, breast, prostate, melanoma and colorectal carcinoma (CRC) and NSCLC ¹⁰. Palliative combination therapy with bevacizumab, a monoclonal antibody against VEGF in patients with previously treated, advanced or metastatic NSCLC has shown promising results in phase II studies with an objective response rate of about 20% and a overall median survival of 12.6 months¹¹. Surprisingly, we do show that overexpression of VEGFA mRNA indicates pronounced response of metastatic lesions towards inhibitors of the EGFR in NSCLC tumors.

Moreover subsequent comparison of VEGF ligand expression with 33 other NSCLC primary tumors revealed a marked overexpression of both VEGFA and VAGFB. Data from head and neck squamous cell carcinoma cell lines, where EGF-R inhibitors lead to downregulation of VEGFA expression casually support our observations ¹². Furthermore, in metastatic CRC a sudden and long lasting reduction of VEGF serum levels was seen during a palliative therapy with cetuximab, an anti-EGF-R antibody¹³. To date, the mechanism(s) leading to VEGF downregulation have not been fully explained. Base on HNSCC cell line experiments it is hypothesized, that VEGF expression might be either downregulated by hypoxia inducible factor 1 alpha (HIF1a), a transcriptional regulator of VEGF expression and/or via the Sp1 binding site at the VEGF promoter^{12,14}. Moreover, it was found that AKT-1 may be able to augment HIF-1alpha expression by increasing its translation under both normoxic and hypoxic conditions. This is in line with our observations in NSCLC tumor tissue that EGFR pathway is able to directly regulate VEGFA and VEGFB expression. The role of VEGF-B is not well studied in this context. Most investigations focus solely on VEGFA, which is named "VEGF" in this context, most probably because of technical insufficiencies of the VEGFB immunhistochemistry detection. However, VEGFB is also capable to bind to VEGFR-2 and VEGFR-2 and potentially has the same activity. Our finding was also surprising as there are conflicting data in squamous cell carcinoma xenografts, where tumours overexpressing VEGFA were resistant to anti-EGF-R based therapy¹⁵. This supports our idea, that a link between EGFR and VEGFR expression exists. However, these reports show that upon development of acquired resistance towards EGFR the VEGFA level increases upon development of resistance. We conclude that basal EGFR expression may be necessary to drive inter alias angiogenesis, stability of newly formed blood vessels andd therby guarantees sufficient supply by nutritients. Elevated basal expression of EGFR or other receptor tyrosin kinases (such as Her-2/neu) after treatment may compensate the deficiency and enable tumor regrowth and/or resistance. However, this is different from the situation that a tumor exhibits an elevated VEGFA expression before treatment and in particular anti-EGFR treatment, indicating a pronounced EGFR activity, which particularly does not necessarily mean "overexpression", being an elementary characteristic of the therapy-naive tumor and thereby resulting in strong EGFR dependence. Also, it has to be stated, that results from the experimental setting like from Viloria-Petit et al¹⁵ often cannot be translated one by one in the in vivo environment of a human cancer, which we have analyzed. We conclude, that EGF-R therapy might have influence on clinical response by downregulation of VEGF-A. In line with this the additional inhibiton of the activity of VEGF ligands and VEGFR activity, as well as related activities such as PDGF ligand and PDGFR activity, may contribute to the anti-tumor effect and be superior to treatment with solely anti-EGFR family or anti VEGF ligand or anti VEGFR or or anti PDGF ligand or anti PDGFR treatments. The specific role of VEGFA, VEGFB , VEGFC and VEGFD in NSCLC and its importance for treatment decisions remains to be investigated in more detail. However, as we show and the overexpression of VEGFA and VEGFB indicates tumors exhibiting dependency towards EGFR activity, which often cannot be directly determined by measuring EGFR expression on RNA and/or protein levels or by determining EGFR mutations. As a first step, NSCLC patients with a good response to erlotinib or gefitinib (another EGF-R tyrosine kinase inhibitor) could be retrospectively evaluated for VEGF gene expression in addition to the presence of EGF-R mutations or gene amplification(s). In a second step the predictive value of VEGF-A expression could be determined in a prospective study. As less than 10% of NSCLC patients will respond to EGF-R inhibitors and therapy with this agent is very costly (30 days of therapy are worth about 2,600 Euro) predictive markers are of utmost importance.

### Example 2

One hundred and six patients with histologically confirmed SCLC were treated from 1989 - 2004 at our university hospital and received ACE as first-line chemotherapy. Histopathological and follow-up data of this cohort were recently published ¹⁷. Formalin fixed paraffin embedded tumour material was available for N=51 patients. Sufficient material for mRNA isolation could be obtained from N=47 cases and those patients were included in this study. Initial staging was performed following the WHO guidelines with CT scans of the thorax and upper abdomen, bronchoscopy and bone scintigraphy. CT scans of the brain were performed at initial diagnosis in the case of limited disease or if clinical examination was suggestive of brain metastases. Additionally, the stage was divided into limited stage disease (LD) and extensive stage disease (ED): limited stage was defined as a disease confined to one hemithorax including mediastinal lymph nodes and/or supraclavivluar lymph nodes. ED was defined by opposition to the criteraia of LD. ACE chemotherapy as first-line therapy was delivered to all 47 patients. Standard ACE chemotherapy, as described by Aisner et al. consists of cyclophosphamide 1,000 mg/m² on day 1, adriamycin (doxorubicin) 45 mg/m² on day 1, and etoposide 100 mg/m² on days 1 to 5 given intravenously every 3 weeks for three cycles¹⁸. Following a response evaluated according to WHO criteria using CT scans and bronchoscopy, chemotherapy was continued for three further cycles in case at least tumour control was possible. Chemotherapy was discontinued earlier in the case of progressive disease, treatment failure, patient refusal or unacceptable toxicity. Dose adjustments were made on the basis of day-1 blood counts and white blood count (WBC) and/or PLT nadir with delay of application and/or dose reduction according to common toxicity criteria (CTC).
Whole brain irradiation (doses between 10x3 Gy or 20x2 Gy) was performed before chemotherapy in 7 ED patients; radiation was given to the mediastinum (doses between 45-55 Gy) in two LD patients. The latter had adjuvant radiation after complete (R0) resection of the upper and the lower lobe, respectively. Additionally, an isolated brain filia (N=2), and a cervical lymph node (N=1) were resected in ED patients before chemotherapy.

### Molecular methods

Two 10 µm thick sections were cut from each paraffin block for molecular analysis of VEGF receptors and ligands. For all tumour samples included in the analysis the number of malignant cells represented at least 50% of all nucleated cells as judged by hematoxylin-eosin staining. VEGF receptor and ligand mRNA was extracted by means of an experimental method based on proprietary magnetic beads from Siemens Medical Solutions. In short, the FFPE slide is deparraffinized in xylol and ethanol, the pellet is washed with ethanol and dried at 55°C for 10 minutes.The pellet is then lysed and proteinized overnight at 55°C with shaking. After adding a binding buffer and the magnetic particles (Siemens Medical Solutions Diagnostic GmbH, Leverkusen, Germany) nucleic acids are bound to the particles within 15 minutes at room temperature. On a magnetic stand the supernatant is taken away and beads can be washed several times with washing buffer. After adding elution buffer and incubating for 10 min at 70°C the supernatant is taken away on a magnetic stand without touching the beads. After normal DNAse I treatment for 30 min at 37°C and inactivation of DNAse I the solution is used for reverse transcription-polymerase chain reaction (RT-PCR).

The quality and quantity of RNA was checked by measuring absorbance at 260 nm and 280 nm. Pure RNA has an A260/A280 ratio of 1.9-2.0. Transcriptional activity of the genes was assessed with quantitative Reverse Transcriptase TaqMan^{™} polymerase chain reaction (RT-PCR) analysis. We applied 40 cycles of nucleic acid amplification and used GAPDH and RPL37A as housekeeping genes at a cycle threshold (CT) of 28. A normalized 2^{(40-ΔCT)} copy number value was calculated that correlates proportionally to RNA transcription levels. For each of the genes RNA-specific Primer/Probe were designed. Expression of each gene was defined as high and low according to values above and up to the median, respectively.

### Statistics

The statistical analysis was performed with SPSS Version 13.0 (SPSS Inc., Chicago, IL). The clinical and biological variables were categorised into normal and pathological values according to standard norms. The Chi-square test was used to compare different groups for categorical variables. To examine correlations between different molecular factors, the Spearman rank correlation coefficient test was used.

For univariate analysis, logistic regression models with one covariate were used when looking at categorical outcomes. The survival curves were estimated by the method of Kaplan and Meier, and the curves were compared according to one factor by the log rank test. For the estimation of multivariate models, all parameters which were significant at the univariate analysis (p<0.05) were fitted to a Cox regression model using a backward forward stepwise method for the selection of covariates. Confidence intervals (CI) at 95% for hazard rates (HR) were calculated. All the probabilities that were calculated were two-tailed.

### Results

### Gene expressions and mutual correlations

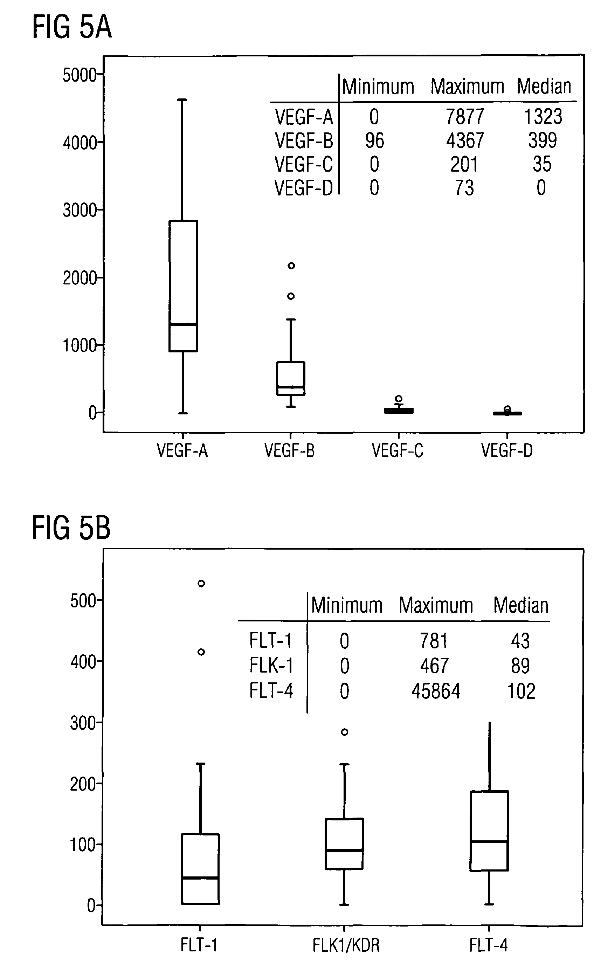

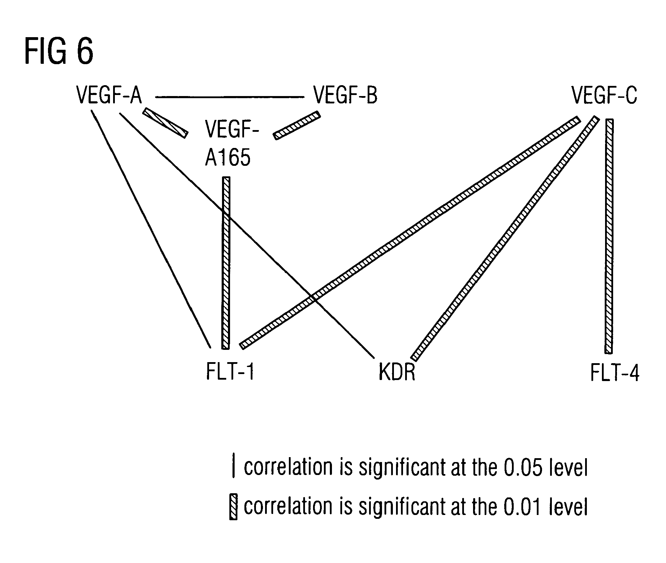

All four members of the VEGF family and all three ligands could be measured by RT-PCR and were expressed to varying extent in the SCLC tumours. VEGF-A, -B, -C and -D gene expression was present in 96%, 100%, 77% and 17% of the cases, respectively. Expression of ligands Flt-1, Flk-1 and Flt-4 was found in 58%, 92% and 87% of the cases, respectively. Distribution and median values of gene expressions are presented in Figs. 5A and B.
Using the Spearman rank correlation coefficient test, it could be shown that VEGF-A, VEGF-A isoform 165 and VEGF-B were significantly linked as well with each other as with their receptor Flt-1. In addition VEGFA was also significantly linked with its receptor KDR, whereas VEGF-C was significantly linked to its receptors KDR and Flt-4. However, but also to Flt-1 (Fig. 6).

Associations of VEGF members and ligands with clinicopathological data
ECOG-PS was positively associated with VEGF-A and VEGF-B. High expression values of VEGFA and VEGFB were indicating better performance status (P=.012 and P=.022). In contrast, high expression of Flt-4 was significantly (P=.022) associated with poor performance status.

Lymph node metastases (N+) was significantly associated with high expression of VEGF-C (P=.046) and pleural effusion was found to be associated with high expression of VEGF-B (P=.049). No significant associations were found between high expression of VEGF members/ligands and gender, age at diagnosis, limited/extended stage disease, weight loss, oxygen saturation (saO₂) and laboratory parameters, e.g. WBC, platelets, LDH or gamma-GT.

Predictive factors for complete response to ACE chemotherapy Complete remission (CR) was found in N=9 (19%) of the patients. Predictive markers for CR were female gender (P=.037) and high expression of VEGF-A (P=.054). Additionally, all patients with CR had high expression values of Flt-1 (P<.001).

### Prognostic factors of survival

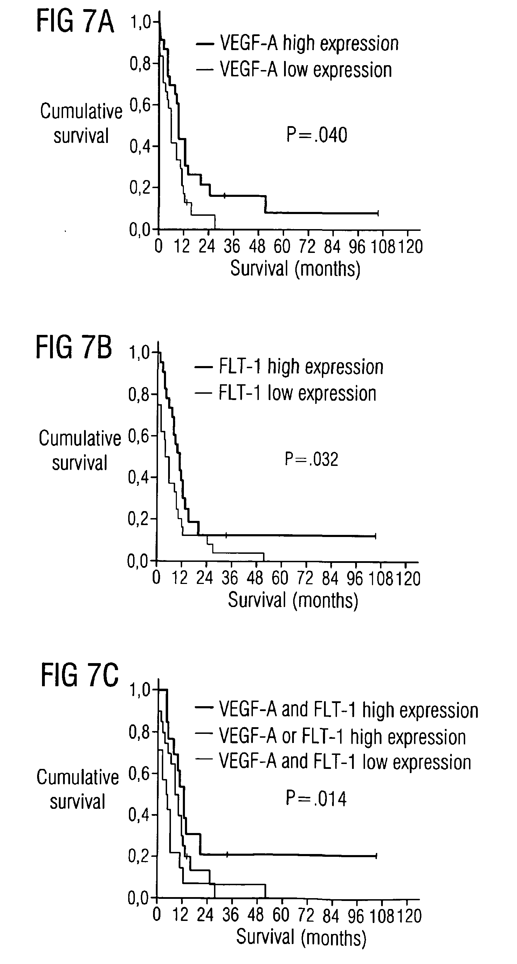

Overall, the estimated median survival time was 8.54 months with 1-, 2- and 5 year survival rates of 30 %, 14% and 4 %, respectively. Prognostic factors, median survival times and their degree of significance are shown in table 1. As we have already described earlier¹⁷, ECOG-PS and LDH were significantly associated with survival, whereas WBC only showed a trend towards significance in this subgroup. Additionally, high expression of both VEGF-A and its ligand Flt-1 were associated with significantly improved survival (Figs. 7A and B). After selection of these parameters for a multivariate Cox regression analysis, ECOG-PS 0 and high Flt-1 expression presented as independent prognostic factors for improved survival (table 2). Interestingly, when taking VEGF-A and Flt-1 expression values as a prognostic marker combination, three highly significant (P=.014) different groups in terms of outcome could be constructed: a favourable group A, consisting of patients with high expression of both VEGF-A and Flt-1 (median survival time 12.6 months); an intermediate group B, consisting of patients with either high expression of VEGF-A or its ligand (median survival time 8.6 months) and finally a poor prognosis group C, consisting of patients with low expression of both VEGF-A and Flt-1 (median survival time 3.9 months). The Kaplan Meier curves are presented in Fig. 7C.

### Discussion

Focusing on VEGF expression in SCLC, we described for the first time gene expression levels of all VEGF family members and their receptors in a large cohort of human tumours. We could show that all VEGF receptors and their ligands are expressed in human SCLC, however to different extent. Our data showing that VEGF-A and Flt-1 gene expression are of positive predictive and prognostic value are surprisingly at first sight. In studies of different kinds of cancer, VEGFA expression is frequently associated with poor outcome and chemotherapy resistance ^{19, 20, 21}. Moreover, a more recently published study of NSCLC tumours describes a significant shorter survival in patients with expression of both VEGF-A and Flt-1²². However, this work refers to patients in the adjuvant setting, not receiving chemotherapy after curative surgery.

In contrast to NSCLC, studies on molecular factors in SCLC are rare. As the VEGF system is concerned, VEGF-A, VEGFR-1, VEGF-C and VEGFR-3 expression was demonstrated in five SCLC cell lines²³. Despite of this, none of the clinical studies on human SCLC focused on the expression of different VEGF family members nor on their receptors ^{24 - 29}. Generally, VEGFA was analyzed by ELISA in serum or by immunohistochemistry in tumor tissue using antibodies against VEGF with different cut off points. Additionally, the chemotherapeutics used in those studies (cisplatin in combination with etoposide, epirubicin or irinotecan) were largely different to the ACE regminen we have applied in this trial. Cyclophosphamid and doxorubicin, as being part of the ACE regimen, have been described to be of significant antiangiogenic value both in vitro and in vivo ³⁰. In the experimental setting dose-response effects of doxorubicin, cyclophosphamide and etoposide on VEGFA 165/164 mediated angiogenesis using the rat mesenteric-window angiogenesis assay were reported^{31, 32}. Doxorubicin and cyclophosphamide significantly suppressed the overall angiogenic response with p values of P<.0002 and P<0.05, respectively. Furthermore, in a murine model, treatment with cyclophosphamide resulted in a significant decrease in the proliferation of both tumour and endothelium ³⁴. Doxorubin response in NSCLC was measured in vitro by Volm et al. ³⁵. Additionally, VEGFA and Flt-1 were immunohistochemically determined, and the expression of these proteins has been compared with the response. The expression of VEGFA and Flt-1 was significantly higher in sensitive tumours than in resistant tumours with p values of P<.001 and P<.01, respectively. The authors hypothesized, that the biologically and therapeutically important hypoxia might had been the reason for the effects of VEGF and its receptor in this study, as some drugs and radiation require oxygen to be maximally cytotoxic ^{35, 36}. It has to be metioned that these immunhistochemistry methods suffer from standardization /reproducibility problems and depend on subjective staining, which impairs the use of these methods for standardized diagnostic purposes.

It has to be mentioned, that these immunhistochemistry methods suffer standardization / reproducibility problems and depend on subjective staining, which corrupt the usage of these methods for standardized diagnostic purposes. Additionally, poor microvessel density is linked with drug resistance ³⁷. The tumour microenvironment provides a complex paracrine network that protects the endothelial compartment. Flt-1 is expressed by tumour -associated endothelial cells (TECs), but not by endothelial cells in adjacent normal adult brain ³⁸. Survival factors, such as VEGFA, secreted by the tumour cells, activate intracellular pathways that prevent TEC apoptosis. VEGFA dose-dependently suppressed proliferation of cultured tumour cells expressing Flt-1 from patients with head and neck cancer ³⁹. Tumours in this experimental setting could not have formed vessels and therefore, Flt-1 receptors expressed on tumour cells could not been directly involved in angiogenesis. Therefore, it is likely that Flt-1 plays an additional non-angiogenic role in direct transduction of VEGF signal to tumour cells. This hypothesis was also confirmed by Zhukova et al., who determinded Flt-1 receptor expression by immunohistochemistry on tumour cells obtained from 83 patients with locally advanced breast cancer after neoadjuvant chemotherapy⁴⁰ . They could show, that patients, whose tumours were expressing Flt-1 had a significantly improved median recurrence-free and survival in contrast to Flt-1 negative tumours.

All these above mentioned data about positive predictive and prognostic value of Flt-1 expression are in line with data presented in our study, obtaining a kind of antiangiogenic therapy in SCLC tumour patients. Flt-1 tumour expression was both a strong predictive factor for CR as well as an independent prognostic factor for median overall survival. Overexpression of both, VEGF and its ligand correlates with the best prognosis in this cohort with median overall survival of 12.6 months, while expression of only one or none of this genes results in intermediate or worst prognosis, respectively. This observation could be explained by different hypothesis. First, Flt1 and VEGFA coexpression in tumour cells could be a good prognostic factor by a negative autocrine feedback loop. Second, as Flt-1 receptors on endothelial cells stimulate the formation of new blood vessels, expression of Flt-1 could be a predictive factor for chemotherapy response by indicating better blood supply, higher proliferative capacity and thereby better access and efficacy of cytotoxic drugs. Third, and contrarly, expression of FLT-1 and VEGFA could indicate hypoxia and stress of the tumor cells, which may sensitze towards additional stress by cytotoxic agents.As VEGFA is the most potent proangiogenic factors, much effort has been directed at the development of agents directly blocking endothelial cell signalling via VEGF receptors and its ligands ⁴¹. Data on NSCLC show effectiveness in combination with chemotherapy. Bevacizumab (Avastin; Roche, Germany) is currently the only clinically available antiangiogenic agent and is licensed for use in combination with 5-fluoroouracil based chemotherapy for first-line treatment of patients with metastatic colorectal carcinoma. However, several antiangiogenic multikinase inhibitors are currently in development, such as sorafenib and sutent and did show promising results in phase two NSCLC trials and currently large phase III trials are ongoing testing the efficacy of these VEGF receptor inhibitors. However, the efficacy of bevacizumab in combination with carboplatin and paclitaxel in recurrent or advanced NSCLC (UICC stages IIIb and IV) has recently been assessed in a phase III study ⁴². The addition of bevacizumab to first-line combination chemotherapy resulted in an increased response rate and a significant survival benefit (median survival 12.3 months vs 10.3 months, P=.003). However, the risk of treatement related deaths - especially bleeding - was increased, when the VEGF antibody was given. The reason, why bevacizumab increases effectiveness of the chemotherapy was postulated in the improvement of drug delivery to the tumour ⁴³. To our knowledge, till now there are no results available about effectiveness of bevacizumab in SCLC. However, as vascularisation plays an important role in SCLC and microvessel density is even higher as in NSCLC, bevacizumab might be effective in this kind of tumour as well ⁴⁴. Additionally, as VEGF-A and Flt-1 tumour gene expressions had been shown to be significantly associated with antiangiogenic ACE therapy in our study, they might be of predictive and prognostic value in bevacizuamb and other antiangiogenic treatment as well. In line with this, we have found strong correlation of EGFR with VEGFR-1, VEGFR-2 and VEGFR-3 expression. In contrast, Her-2/neu expression correlated with VEGFR-2 and VEGFR-3 to some extent. Therefore the observations of example 1, that VEGF overexpressing tumours may particularly benefit from anti EGFR family therapies related to potential response of these tumors to anti-ErbB family regimens is also reasonable in SCLC. We further conclude, that this is a general tumour mechanism to be found in almost all other types of cancers, malignancies and transformations associated with the lung, ovarian, cervix, esophagus, stomach, pancreas, prostate, head and neck, renal cell, colon or breast. Further retrospective studies should be performed, to test these hypothesis to warrant subsequent prospective trial designs.

In table 6, primer sequences (forward primer and reverse primer) reverse and probe sequences used in accordance with the present invention are shown. These sequences can be DNA, RNA, PNA, LNA and/or Morpholino. As for the probe sequences, the latter refer to sequences which are labeled with a fluorescent marker at one end and a quencher of fluorescence at the opposite end of the probe. The close proximity of the reporter to the quencher prevents detection of its fluorescence; breakdown of the probe by the 5' to 3' exonuclease activity of the taq polymerase breaks the reporter-quencher proximity and thus allows unquenched emission of fluorescence, which can be detected. An increase in the product targeted by the reporter probe at each PCR cycle therefore causes a proportional increase in fluorescence due to the breakdown of the probe and release of the reporter.

### Figures

Figures 1 - 4: Radiological images and molecular analysis as referred to in example 1

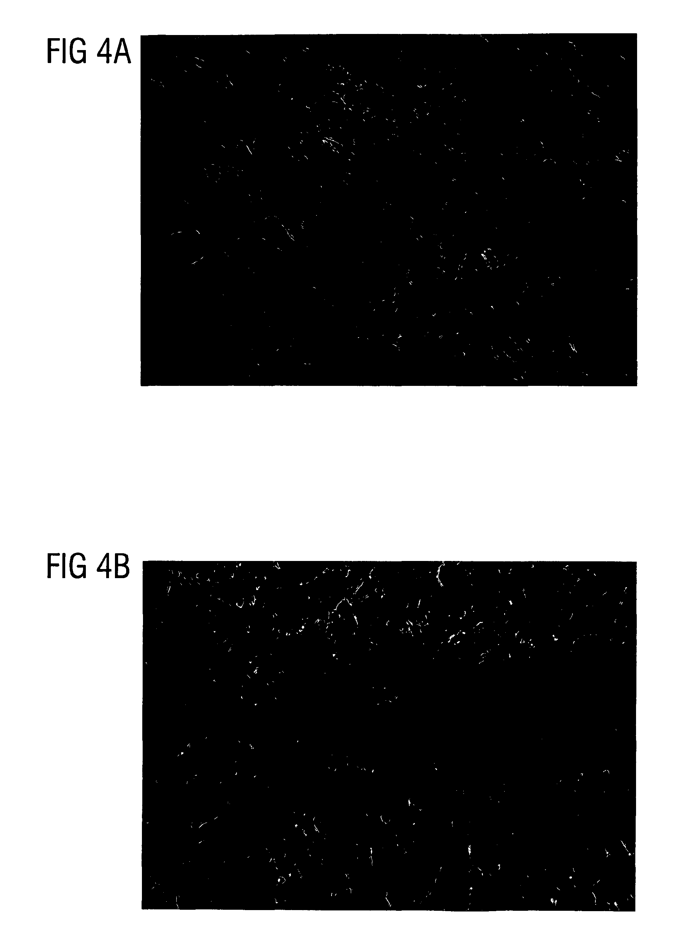

Contrast enhanced computed tomography scans before starting Erlotinib therapy shows a primary tumor in the left lower lobe of the lung (Fig. 1A) and multiple liver metastases (Fig. 2A) and 4 months later after confirmed partial response with diminshed primary lung tumour (Fig. 1B) and decreased liver metastases (Fig. 2B). Cromogenic in situ hybridisation (CiSH) analysis shows low polysomy of the EGF-R locus in the nuclei of the tumour cells (Fig. 3A) . Expression analysis of EGF-R and VEGF genes in normal lung epithilium, primary tumor and liver metastases, shows overexpression of VEGF-A in the tumour (Fig. 3B). Immunohistochemistry with antibodies against VEGF-A shows a strong cytoplasmic staining as well in the primary lung tumour (Fig. 4A) as in the liver metastasis (Fig. 4B). The remaining figures are described in the context of the respective examples.

### Figure 5A, B: Gene expressions and mutual correlations

All four members of the VEGF family and all three ligands could be measured by RT-PCR and were expressed to varying extent in the SCLC tumours. VEGF-A, -B, -C and -D gene expression was present in 96%, 100%, 77% and 17% of the cases, respectively. Expression of ligands Flt-1, Flk-1 and Flt-4 was found in 58%, 92% and 87% of the cases, respectively. Distribution and median values of gene expressions are presented in Figures 5A and B.

### Figure 6: Correlation between different factors and receptors

Using the Spearman rank correlation coefficient test, it could be shown that VEGF-A, VEGF-A isoform 165 and VEGF-B were significantly linked as well with each other as with their receptor Flt-1. In addition VEGFA was also significantly linked with its receptor KDR, whereas VEGF-C and VEGF-D wereas significantly linked as well to each other as witht its receptors KDR and Flt-4. However, but VEGF-C was also linked to Flt-1

### Figure 7: median survival times

High expression of both VEGF-A and its ligand Flt-1 were associated with significantly improved survival (Figs. 7A and B). After selection of these parameters for a multivariate Cox regression analysis, ECOG-PS 0 and high Flt-1 expression presented as independent prognostic factors for improved survival. Interestingly, when taking VEGF-A and Flt-1 expression values as a prognostic marker combination, three highly significant (P=.014) different groups in terms of outcome could be constructed: a favourable group A, consisting of patients with high expression of both VEGF-A and Flt-1 (median survival time 12.6 months); an intermediate group B, consisting of patients with either high expression of VEGF-A or its ligand (median survival time 8.6 months) and finally a poor prognosis group C, consisting of patients with low expression of both VEGF-A and Flt-1 (median survival time 3.9 months). The Kaplan Meier curves are presented in Fig. 7C.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

**Table 1: Univariate survival analysis in SCLC patients (N=47) treated with ACE chemotherapy**

| Factors | | Median survival time (months) | STE | 95% CI | Significance * |
|---|---|---|---|---|---|
| Gender | | | | | |
| | Male | 5.98 | 1.964 | 2.131 - | n.s. |
| | | | | 9.828 | |
| | Female | 11.11 | 2.401 | 6.398 - | |
| | | | | 15.812 | |
| ECOG-PS | | | | | |
| | 0 | 12.19 | 3.276 | 5.769 - | <.001 |
| | | | | 18.608 | |
| | 1/2 | 4.40 | 1.562 | 1.341 - | |
| | | | | 7.464 | |
| Stage | | | | | |
| | Limited | 5.82 | 2.137 | 1.626 - | n.s. |
| | | | | 10.004 | |
| | Extended | 8.81 | 2.236 | 4.226 - | |
| | | | | 12.990 | |
| WBC | | | | | |
| | Normal | 9.60 | 1.024 | 7.587 - | .082 |
| | | | | 11.600 | |
| | Elevated | 4.27 | 0.514 | 3.263 - | |
| | | | | 5.279 | |
| LDH | | | | | |
| | Normal | 9.76 | 2.146 | 5.552 - | .030 |
| | | | | 13.963 | |
| | Elevated | 5.98 | 0.219 | 5.551 - | |
| | | | | 6.408 | |
| VEGF-A | | | | | |
| | Low expression | 5.82 | 1.086 | 3.686 - | .040 |
| | | | | 7.945 | |
| | High expression | 9.73 | 0.289 | 9.156 - | |
| | | | | 10.290 | |
| VEGF-B | | | | | |
| | Low expression | 5.75 | 1.128 | 3.538 - | n.s. |
| | | | | 7.961 | |
| | High | 9.40 | 0.785 | 7.858 - | |
| expression | | | | 10.934 | |
| VEGF-C | | | | | |
| | Low expression | 5.75 | 2.153 | 1.530 - | n.s. |
| | | | | 9.969 | |
| | High | 9.76 | 2.046 | 5.747 - | |
| expression | | | | 13.769 | |
| FLT-1 | | | | | |
| | Low Expression | 4.24 | 1.147 | 1.990 - | .032 |
| | | | | 6.486 | |
| | High | 11.00 | 1.364 | 8.332 - | |
| Expression | | | | 13.680 | |
| FLT-4 | | | | | |
| | Low Expression | 8.54 | 2.314 | 4.007 - | |
| | | | | 13.077 | |
| | High | 8.44 | 2.702 | 3.147 - | n.s. |
| Expression | | | | 13.740 | |

| | | | | | |
|---|---|---|---|---|---|
| * log rank analysis p<.05 | | | | | |

**Table 2: Multivariate Cox regression analysis for survival factors in SCLC patients**

| Factors | Coefficient | STD | HR (95% P value CI) |
|---|---|---|---|
| ECOG-PS (0 vs. 1/2) | 1.776 | 5.908 | (1.975 - .002 |
| | | | 17.839) |
| LDH (normal vs | 0.105 | 1.111 | (0.693 - .662 |
| elevated) | | | 1.780) |
| VEGF-A (low vs high - | 0.575 | 0.562 | (0.264 - .137 |
| expression) | | | 1.200) |
| FLT-1 (low vs - | 1.227 | 0.293 | (0.133 - .002 |
| high expression) | | | 0.644) |

**Table 3: Genes of Interest**

| Gene_ | Ref. | Sequences | Ref. Sequences | Locus_Li nk_ID | Unige ne_ID | OMIM |
|---|---|---|---|---|---|---|
| Symbol [A] | Description | [A] | [A] | [A] | [A] | [A] |
| | Vascular | | | | | |
| | endothelial | growth | NM_00337 | | | |
| VEGFA | factor | | 6 | 7422 | 73793 | 192240 |
| | Vascular | | | | | |
| | endothelial | growth | NM_00337 | | | |
| VEGFB | factor B | | 7 | 7423 | 78781 | 601398 |
| | Vascular | | | | | |
| | endothelial | growth | | | | |
| | factor | C | NM_00542 | | | |
| VEGFC | preproprotein | | 9 | 7424 | 79141 | 601528 |
| FIGF / | Vascular | | NM_00446 | | 11392 | 300091 |
| VEGFD | endothelial | growth | 9 | | | |
| | factor D | | | | | |
| | preproprotein | | | | | |
| | epidermal | growth | | | | |
| | factor receptor | | | | | |
| | (erythroblastic | | | | | |
| | leukemia viral (v- | | | | | |
| EGFR / | erb-b) oncogene | | NM_00522 | | | |
| HER-1 | homolog, avian) | | 8 | 1956 | 77432 | 131550 |
| | v-erb-b2 | | | | | |
| | erythroblastic | | | | | |
| | leukemia viral | | | | | |
| | oncogene homolog 2, | | | | | |
| ERBB2 / | neuro/glioblastoma | | | | | |
| Her-2/neu | derived oncogene | | NM_00444 | | 32391 | |
| / HER-2 | homolog | | 8 | 2064 | 0 | 164870 |
| | v-erb-b2 | | | | | |
| | erythroblastic | | | | | |
| ERBB3 / | leukemia viral | | NM_00198 | | 19906 | |
| HER-3 | oncogene homolog 3 | | 2 | 2065 | 7 | 190151 |
| | v-erb-a | | | | | |
| | erythroblastic | | | | | |
| ERBB4 / | leukemia viral | | NM_00523 | | | |
| HER-4 | oncogene homolog 4 | | 5 | 2066 | 1939 | 600543 |
| | Vascular | | | | | |
| FLT-1 / | endothelial | growth | NM_00201 | | 65436 | |
| VEGFR-1 | factor recetor | | 9.2 | | 0 | 165070 |
| | fms-related | | NM_00411 | | | |
| FLT-3 | tyrosine kinase 3 | | 9 | 2322 | 385 | 136351 |
| FLT-4 / | fms-related | | NM_00202 | | | |
| VEGFR-3 | tyrosine kinase 4 | | 0 | 2324 | 74049 | 136352 |
| KDR / | kinase insert | | NM_00225 | 3791 | 12337 | 191306 |
| VEGFR-2 | domain receptor (a | | 3 | | | |
| | type III receptor | | | | | |
| | tyrosine kinase) | | | | | |
| | platelet-derived | | | | | |
| | growth factor alpha | | NM_00260 | | | |
| PDGFA | isoform 1 | | 7 | 5154 | 37040 | 173430 |
| | platelet-derived | | | | | |
| | growth factor beta | | | | | |
| | isoform 1, | | NM_00260 | | | |
| PDGFB | preproprotein | | 8 | 5155 | 1976 | 190040 |
| | platelet-derived | | | | | |
| | growth factor C | | NM_01620 | | | |
| PDGFC | precursor | | 5 | 56034 | 43080 - | |
| | platelet-derived | | | | | |
| | growth factor | | | | | |
| | receptor alpha | | NM_00620 | | 16607 | |
| PDGFRA | precursor | | 6 | 5156 | 4 | 173490 |
| | platelet-derived | | | | | |
| | growth factor | | | | | |
| | receptor alpha | | NM_00620 | | | |
| PDGFRA | precursor | | 6 | 5156 | 74615 | 173490 |
| | platelet-derived | | | | | |
| | growth factor | | | | | |
| | receptor beta | | NM_00260 | | | |
| PDGFRB | precursor | | 9 | 5159 | 76144 | 173410 |
| | platelet-derived | | | | | |
| | growth factor | | | | | |
| | receptor-like | | NM_00620 | | 17004 | |
| PDGFRL | protein | | 7 | 5157 | 0 | 604584 |

The terms "Ref. Sequences, Locus_Link_ID, Unigene_ID and OMIM" relate to databases in which the respective proteins are listed under the given access number. These databases can be accessed over the NCBI server.

**Table 4: Predictive factors for complete remission (N=9) in SCLC patients treated with ACE chemotherapy**

| Factors | | Cases | Complete (CR) | Remission | P value * |
|---|---|---|---|---|---|
| | | | N | (%) | |
| Gender | | | | | |
| | Male | 34 | 4 | (11.8) | .037 |
| | Female | 13 | 5 | (38.5) | |

| ECOG-PS | | | | | |
|---|---|---|---|---|---|
| | 0 | 18 | 5 | (27.8) | n.s. |
| | 1/2 | 29 | 4 | (13.8) | |

| Stage | | | | | |
|---|---|---|---|---|---|
| | Limited | 8 | 3 | (37.5) | n.s. |
| | Extendede | 39 | 6 | (15.4) | |

| WBC | | | | | |
|---|---|---|---|---|---|
| | Normal | 27 | 6 | (22.2) | n.s. |
| | Elevated | 20 | 3 | (15.0) | |

| LDH** | | | | | |
|---|---|---|---|---|---|
| | Normal | 13 | 4 | (30.8) | n.s. |
| | Elevated | 25 | 3 | (12.0) | |

| VEGF-A | | | | | |
|---|---|---|---|---|---|
| | Low | 24 | 2 | (8.3) | .054 |

| expression | | | | | |
|---|---|---|---|---|---|
| | High | 23 | 7 | (30.4) | |

| expression | | | | | |
|---|---|---|---|---|---|
| VEGF-B | | | | | |
| | Low | 23 | 6 | (26.1) | n.s. |

| expression | | | | | |
|---|---|---|---|---|---|
| | High | 24 | 3 | (12.5) | |

| VEGF-C | | | | | |
|---|---|---|---|---|---|
| | Low | 23 | 5 | (20.8) | n.s. |

| expression | | | | | |
|---|---|---|---|---|---|
| | High | 24 | 4 | (17.4) | |

| expression | | | | | |
|---|---|---|---|---|---|
| FLT-1 | | | | | |
| | Low | 24 | 0 | (0) | 0.001 |

| expression | | | | | |
|---|---|---|---|---|---|
| | High | 23 | 9 | (100) | |

| expression | | | | | |
|---|---|---|---|---|---|
| FLT-4 | | | | | |
| | Low | 24 | 5 | (56) | |

| expression | | | | | |
|---|---|---|---|---|---|
| | High | 23 | 4 | (44) | n.s. |
| expression | | | | | |

| | | | | | |
|---|---|---|---|---|---|
| * Chi-square Test <.05; ** LDH not determined in N=9 patients | | | | | |

**Table 5: Clinopathologic findings in N = 47 SCLC tumor patients treated with ACE first line chemotherapy**

| **Factors** | | **Cases** | **VEGF-A** | | | **VEGF-B** | | | **VEGF-C** | | | **FLT-4** | | |
|---|---|---|---|---|---|---|---|---|---|---|---|---|---|---|
| | | N | N | % | P | N | % | P | N | % | P | N | % | P |
| Gender | | | | | | | | | | | | | | |
| | Male | 34 | 18 | (53) | | 18 | (53) | | 16 | (47) | | 16 | (47) | |
| | Female | 13 | 5 | (38) | n.s. | 6 | (46) | n.s. | 7 | (54) | n.s. | 7 | (54) | n.s. |

| ECOG | | | | | | | | | | | | | | |
|---|---|---|---|---|---|---|---|---|---|---|---|---|---|---|
| | 0 | 18 | 13 | (72) | | 13 | (72) | | 10 | (56) | | 5 | (28) | |
| | 1/2 | 29 | 10 | (34) | **.012** | 11 | (38) | **.022** | 13 | (45) | | 18 | (62) | **.022** |

| Stage | | | | | | | | | | | | | | |
|---|---|---|---|---|---|---|---|---|---|---|---|---|---|---|
| | Limited | 8 | 4 | (50) | | 5 | (62) | | 6 | (75) | | 4 | (50) | |
| | Extended | 39 | 19 | (49) | n.s. | 19 | (49) | n.s. | 17 | (44) | n.s. | 19 | (49) | n.s. |

| N classification | | | | | | | | | | | | | | |
|---|---|---|---|---|---|---|---|---|---|---|---|---|---|---|
| | NO | 7 | 3 | (43) | | 3 | (43) | | 1 | (14) | | 3 | (43) | |
| | N + | 40 | 19 | (50) | n.s. | 21 | (55) | n.s. | 21 | (55) | **.046** | 20 | (53) | n.s. |

| Pleural | | | | | | | | | | | | | | |
|---|---|---|---|---|---|---|---|---|---|---|---|---|---|---|
| effusion | | | | | | | | | | | | | | |
| | No | 29 | 15 | (52) | | 6 | (33) | | 8 | (44) | | 11 | (61) | |
| | Yes | 18 | 8 | (44) | n.s. | 18 | (62) | **.049** | 15 | (52) | n.s. | 12 | (41) | n.s. |

| O₂ Saturation | | | | | | | | | | | | | | |
|---|---|---|---|---|---|---|---|---|---|---|---|---|---|---|
| | normal | 20 | 11 | (55) | | 9 | (45) | | 9 | (45) | | 10 | (50) | |
| | < 60mm Hg | 10 | 6 | (60) | n.s. | 4 | (40) | n.s. | 5 | (50) | n.s. | 6 | (60) | n.s. |

**Table 6: primer sequences and probe sequences used in accordance with the present invention**

| SEQ ID | Gene | PCR probe | Forward primer | Reverse primer |
|---|---|---|---|---|
| 1-3 | VEGFA | cacattgttggaagaa gcagcccatgac | cagatgtcccggcgaag a | Gagggcgagtcccaggaa |
| 4-6 | VEGFA | cgttcgtttaactcaa gctgcctcg | aacacagactcgcgttg caa | Cggcttgtcacatctgcaa gt |
| 7-9 | VEGFA | aacttcctcgggttca taaccatagcagtcc | cccccaacatctggtta gtctt | Ccacgggcacagaatatgc |
| 10-12 | VEGFA | caccatgcagattatg cggatcaaacct | gcccactgaggagtcca aca | Tcctatgtgctggccttgg t |
| 13-15 | VEGFA Isoform 121 | caccatgcagattatg cggatcaaacct | gcccactgaggagtcca aca | Gcctcggcttgtcacattt t |
| 16-18 | VEGFA Isoform 165 | caccatgcagattatg cggatcaaacct | gcccactgaggagtcca aca | Agcaaggcccacagggatt t |
| 19-21 | VEGFA Isoform 185 | caccatgcagattatg cggatcaaacct | gcccactgaggagtcca aca | aacgctccaggacttatac cg |
| 22-24 | VEGFB | acaggctgccactcc ccacc | aatgcagacctaaaaaa aaggacagt | Cccagcccggaacagaa |
| 25-27 | VEGFB | cacatctatccatgac accactttcctctgg | tggcaggtagcgcgagt at | Ccctgtctcccagcctgat |
| 28-30 | VEGFB | ttcctcccctcactaa gaagacccaaacct | ccactctgtgcaagtaa gcatctt | Gtaccaaagcccaaatccc att |
| 31-33 | VEGFD | tgacattgaaacacta aaagttatagatgaag aatggca | actaggtttgcggcaac tttct | Tctctagggctgcactgag ttct |
| 34-36 | VEGFC | acggccatgtacgaac cgcca | gttccaccaccaaacat gca | Cactatatgaaaatcctgg ctcaca |
| 37-39 | VEGFC | aaacatggcccggcgt | ccagaatagaagtcatg | Tttagatcagagcaaatgt |
| | | caacc | ctttgatg | cttgca |
| 40-42 | VEGFC | ttgagtcatctccagc atccgaggaaa | ccacagatgtcatggaa tccat | Tgcctggctcaggaagatt t |
| 43-45 | VEGFC | agaacaggccaacctc aactcaaggacag | gagatccccatggaggt cttc | Cactcattatcaatacttt tcaagatctctgt |
| 46-48 | VEGFC | tgcatgccacgggagg tgtgtataga | aatagaccctggagtga aaccatt | Tattgcagcaacccccaca t |
| 49-51 | VEGFC | acatgcagctgttaca gacggccatgt | ctgagcaagcggtctct gagt | Cactatatgaaaatcctgg ctcaca |
| 52-54 | VEGF-D | ccatcctctaccagaa catacatcagttattt ggaga | cccttcccaccaagtgt tca | Tggtgctgcctcactggat |
| 55-57 | FLT-1 (VEGFR1) | ccaggccatgatggcc ttacactga | ctgaaaacttagccaga gttaggttgt | Cagaataataccaagaaat tgagttaagtgtct |
| 58-60 | FLT-1 (VEGFR1) | atcccaagaaacctcc ccagacctt | tgcatcacgtagggtga cttct | Gagccagacttctcccatg gt |
| 61-63 | FLT-1 (VEGFR1) | tgctgtcgccctggta gtcatcaaaca | catgggagaggccaaca ga | Aacctttgaagaacttttta ccgaatg |
| 64-66 | KDR, (VEGFR2) | ttcttggcatcgcgaa agtgtatccac | tgttacagcttccaagt ggctaag | Atttttaaccacgttcttct ccgataa |
| 67-69 | FLT4 (VEGFR3) | cagtggcaatacggag gcaaccg | ttaacctctgtgtgcta gctttctatct | Cctacgcatgtgtgcattc c |
| 70-72 | FLT4 (VEGFR3) | tgcctgcttccctggg tagtccc | gcacccacttaccccgc | Gagtttaactcaggtgtca cctttga |
| 73-75 | FLT4 (VEGFR3) | aagggattttcatgca caatgacctcgg | tgagccactcgacgctg at | Cagcgatttcgggagagc |
| 76-78 | FLT4 (VEGFR3) | aagaaagcggcttcag ctgtaaaggacct | tgagcagatagagagca ggcatag | Ccctggtcacagccacatt c |
| 79-81 | FLT4 (VEGFR3) | aggcatagacaagaaa gcggcttcaggta | ctcggaggagtttgagc agatag | Ctgacgtatgctgccttct ctct |

### References

- 1.: Parkin DM, Lancet Oncol 2001;2(9):533-43.
- 2.: Shepherd FA, N Engl J Med 2005;353(2):123-32.
- 3.: Perez-Soler R, J Clin Oncol 2004;22(16):3238-47.
- 4.: Tsao MS, N Engl J Med 2005;353(2):133-44.
- 5.: Pao W, J Clin Oncol 2005;23(11):2556-68.
- 6.: Tokumo M, Lung Cancer 2006;53(1):117-21.
- 7.: Giaccone G, Clin Cancer Res 2006;12(20 Pt 1): 6049-55.
- 8.: Cappuzzo F, J Natl Cancer Inst 2005;97(9):643-55.
- 9.: Hicklin DJ, J Clin Oncol 2005;23(5):1011-27.
- 10.: Langer CJ, Semin Oncol 2005;32(6 Suppl 10):S23-9.
- 11.: Sandler A, Clin Cancer Res 2006;12(14 Pt 2):4421s-4425s.
- 12.: Pore N, Cancer Res 2006;66(6):3197-204.
- 13.: Vincenzi B, Ann Oncol 2006;17(5):835-41.
- 14.: Luwor RB, Oncogene 2005;24(27):4433-41.
- 15.: Viloria-Petit A, Cancer Res 2001;61(13):5090-101.
- 16.: Murray S, Lung Cancer 2006; 52:225-33.
- 17.: Brueckl-WM, AnticancerRes 206:4825, 2006
- 18.: Aisner-J, Cancer Chemother Pharmacol 7:187, 1982
- 19.: Brown-LF, Cancer Res 53:4727, 1993
- 20.: Brown-LF HumPathol 26:86, 1995;
- 21.: Fontanini-G, BrJCancer 75:1295, 1997
- 22.: Seto-T, LungCancer 53:91, 2006
- 23.: Tanno et al., LungCancer 46:11, 2004
- 24.: Lucchi-M, EurJCardiothor Surg 21:1105, 2002
- 25.: Dowell-JE, Anticancer Res 24:2367, 2004
- 26.: Hasegawa-Y, IntMed 44:26, 2005
- 27.: Tas-F, Cancer Invest 24:492, 2006
- 28.: Fontanini-G, BrJCancer 86:558, 2002
- 29.: Salven-P, IntJCancer 79:144, 1998
- 30.: Miller KD, Journal of Clinical Oncology 19:1195-1206, 2001
- 31.: Lennernäs-B et al., ActaOncologica 42:294, 2003
- 32.: Per et al., APMIS 11:995-1003, 2003
- 33.: Oken, M.M., Am J Clin Oncol 5:649-655, 1982.
- 34.: Bassukas-ID et al, Virchows Arch B Cell Pathol Incl Mol Methol 59:3776, 1999.
- 35.: Volm-M et al., AnticancerRes 17:99, 1997.
- 36.: Teichler-BA, Cancer Metast Rev 13:139, 1994.
- 37.: Koomägi R, Mattern-J et al., Carcinogenesis 16:2129, 1995
- 38.: Plate-K et al, Int JCancer 59:520, 1994.
- 39.: Herold-Mende et al. LabInvest 79:1573, 1999
- 40.: Zhukova et al., Bull Exp Biol Med 5:478,
- 41.: Sandler, Sem Oncol 32(suppl) 2005.
- 42.: Sandler-A, NEJM 355:2542, 2006.
- 43.: Willett-CG et al., NatMed 10:145, 2004
- 44.: Stefanou-D et al., Histol Histopathol 19:37, 2004.

## Claims

1. A method for predicting a clinical response of a patient suffering from or at risk of developing a neoplastic disease towards a given mode of treatment, said method comprising the steps of:
a) obtaining a biological sample from said patient;
b) determining the expression level of at least one ligand from the Vascular endothelial growth factor (VEGF) family and/or of at least one receptor from the Vascular endothelial growth factor receptor (VEGFR) family in said patient,
c) comparing the pattern of expression levels determined in (b) with one or several reference pattern(s) of expression levels; and
d) predicting therapeutic success for said given mode of treatment in said subject or implementing therapeutic regimen targeting the signalling pathway said ligand is related to in said subject from the outcome of the comparison in step (c).

2. The method according to claim 1, **characterized in that** the mode of treatment for which prediction is sought is a treatment related to the signalling pathway of receptors from the ErbB receptor family.

3. The method according to claim 1, **characterized in that** the mode of treatment for which prediction is sought is a treatment related to the signalling pathway of a ligand from the VEGF family and/or of a receptor from the VEGFR family.

4. The method according to any one of claims 1 to 3, said method further comprising the steps of:
a) obtaining a biological sample from a patient;
b) determining the pattern of expression levels of at least one gene selected from the Vascular endothelial growth factor (VEGF) or Vascular endothelial growth factor receptor (VEGFR) family in said patient,
c) comparing the pattern of expression levels determined in (b) with one or several reference pattern(s) of expression levels;
wherein upregulated expression of at least one ligand determined in (b) is indicative of a promising prediction as regards therapeutic success for a mode of treatment related to the signalling pathway of receptors from the ErbB receptor family.

5. The method according to any one of claims 1 to 4, wherein said given mode of treatment (a) acts on recruitment of lymphatic vessels, cell proliferation, cell survival and/or cell motility, and/or , and/or b) comprises administration of a chemotherapeutic agent.

6. The method according to any of claims 2 - 5, **characterized in that**
a) at least one of the said genes the expression level of which is determined is VEGF-A, VEGF-B, VEGFR-1 or VEGFR-2 and/or
b) the mode of treatment for which prediction is sought is a treatment related to the signalling pathway of EGFR.

7. The method according any of claims 2 - 6, wherein said given mode of treatment comprises chemotherapy, administration of small molecule inhibitors, antibody based regimen, anti-proliferation regimen, pro-apoptotic regimen, pro-differentiation regimen, radiation and/or surgical therapy.

8. A method of selecting a therapy modality for a patient afflicted with a neoplastic disease, said method comprising the steps of:
a) obtaining a biological sample from said patient;
b) predicting from said sample, by the method according to any one of claims 1 to 6, therapeutic success for a plurality of individual modes of treatment; and
c) selecting a mode of treatment which is predicted to be successful in step (b).

9. The method according to claim 8, comprising
a) obtaining a sample comprising cancer cells from said patient;
b) separately maintaining aliquots of the sample in the presence of one or more test compositions;
c) comparing expression of a single or plurality of molecules, selected from the ligands and/or receptors listed in table 3 in each of the aliquots; and
d) selecting a test composition which induces a lower level of expression of ligands and/or receptors from table 3 and/or a higher level of expression of ligands and/or receptors from table 3 in the aliquot containing that test composition, relative to the level of expression of each ligand in the aliquots containing the other test compositions.

10. The method according to any one of claims 1 to 8, wherein the expression level is determined by
a) a hybridization based method;
b) a PCR based method;
c) determining the protein level,
d) a method based on the electrochemical detection of particular molecules, and/or by
e) an array based method.

11. The method according to any one of claims 1 to 10, wherein said cancer or neoplastic disease is select from the group consisting of small cell lung cancer (SCLC), non small cell lung cancer (NSCLC), and/or transformations associated with the ovary, cervix, esophagus, stomach, pancreas, prostate, head and neck, renal cell, colon and/or breast.

12. The method to any one of claims 1 to 11, **characterized in that** the expression level of at least one of the said ligands is determined with rtPCR (reverse transcriptase polymerase chain reaction) of the ligand related mRNA.

13. The method according to any of claims 1 - 12, **characterized in that** the expression level of at least one of the said ligands of is determined in formalin and/or paraffin fixed tissue samples.

14. The method according to any one of claims 1 - 13, wherein, after lysis, the samples are treated with silica-coated magnetic particles and a chaotropic salt, in order to purify the nucleic acids contained in said sample for further determination.

15. The method according to any of claims 1 - 14, wherein said treatment related to the signalling pathway of receptors from the ErbB receptor family comprises the administration of an agonist of the ErbB receptor domain.

16. The method according to any of claims 1 - 15, wherein said treatment related to the signalling pathway of receptors from the ErbB receptor family comprises the administration of a tyrosin kinase inhibitor.

17. The method according to any of claims 1 - 16, wherein said treatment related to the signalling pathway of receptors from the ErbB receptor family comprises the administration of chemotherapeutics.

18. A kit useful for carrying out a method of any one of claims 1 to 17, comprising at least
a) a primer pair and/or a probe each having a sequence sufficiently complementary to a gene encoding for a ligand from the VEGF family and/or a receptor from the VEGFR family and/or
b) at least an antibody directed against a ligand from the VEGF family and/or a receptor from the VEGFR family and/or

19. Use of
a) an antibody directed against a ligand from the VEGF family and/or a receptor from the VEGFR family,
b) an antisense nucleic acid or a ribozyme inhibiting the expression of a Gene encoding for a ligand from the VEGF family and/or for a receptor from the VEGFR family, or
c) an inactive version of a ligand from the VEGF family and/or of a receptor from the VEGFR family,
as an antagonist for the preparation of a pharmaceutical composition for the treatment of a cancer associated with the signalling pathway of receptors from the ErbB receptor family.

20. A method for correlating the clinical outcome of a patient suffering from or at risk of developing a neoplastic with the presence or non-presence of a defect in ErbB receptor expression, said method comprising the steps of:
a) obtaining a fixed biological sample from said patient;
b) determining the expression level of at least one ligand from the VEGF family and/or one receptor from the VEGFR family in said patient according to any of the above methods, and
c) correlating the pattern of expression levels determined in (b) with said patient's data, said data being selected from the group consisting of etiopathology data, clinical symptoms, anamnesis data and/or data concerning the therapeutic regimen.

21. A nucleic acid molecule, selected from the group consisting of
a) the nucleic acid molecule presented as SEQ ID NO:1 - 81
b) a nucleic acid molecule having a length of 4 - 80 nucleotides, preferably 18 - 30 nucleotides, the sequence of which corresponds to the sequence of a single stranded fragment of a gene encoding for a ligand and/or receptor selected from the group consisting of VEGFA, VEGFB, VEGFC, FIGF / VEGFD, EGFR / HER-1, ERBB2 / Her-2/neu / HER-2, ERBB3 / HER-3, ERBB4 / HER-4, FLT-1 / VEGFR-1, FLT-3, FLT-4 /VEGFR-3, KDR / VEGFR-2, PDGFA, PDGFB, PDGFC, PDGFRA, PDGFRA, PDGFRB and/or PDGFRL,
c) a nucleic acid molecule that is a fraction, variant, homologue, derivative, or fragment of the nucleic acid molecule presented as SEQ ID NO: 1 - 81
d) a nucleic acid molecule that is capable of hybridizing to any of the nucleic acid molecules of a) - c) under stringent conditions
e) a nucleic acid molecule that is capable of hybridizing to the complement of any of the nucleic acid molecules of a) - d) under stringent conditions
f) a nucleic acid molecule that is capable of hybridizing to the complement of a nucleic acid molecule of e
g) a nucleic acid molecule having a sequence identity of at least 95 % with any of the nucleic acid molecules of a) - f)
h) a nucleic acid molecule having a sequence identity of at least 70 % with any of the nucleic acid molecules of a) - f) i) a complement of any of the nucleic acid molecules of a) - h), or
i) a nucleic acid molecule that comprises any nucleic acid molecule of a) - i).

22. The nucleic acid according to claim 21, **characterized in that** the said nucleic acid is selected from the group consisting of DNA, RNA, PNA, LNA and/or Morpholino

23. The nucleic acid according to any of claims 21 - 22, **characterized in that** it is labelled with at least one detectable marker.

24. A kit of primers and/or detection probes, comprising at least one of the nucleic acids according to any of claims 21 - 23 and/or their fractions, variants, homologues, derivatives, fragments, complements, hybridizing counterparts, or molecules sharing a sequence identity of at least 70%, preferably 95 %.

25. The kit according to claim 24, comprising at least one of the nucleic acid molecules presented as SEQ ID NO: 1 - 81, and/or their fractions, variants, homologues, derivatives, fragments, complements, hybridizing counterparts, or molecules sharing a sequence identity of at least 70%, preferably 95 %, for the detection of at least one ligand from the VEGF family and/or at least one receptor from the VEGFR family.

26. The kit according to claim 24, comprising at least one of the nucleic acid molecules presented as SEQ ID NO: 1 - 21, for the detection of different isoforms of VEGFA.

27. Use of a nucleic acid according to any of claims 21 - 23 or of a kit according to any of claims 24 - 26 for predicting a clinical response of a patient suffering from or at risk of developing a neoplastic disease towards a given mode of treatment.


SEQUENCE LISTING

<110>  Siemens AG

<120>  A method for predicting a clinical response of a patient
       suffering from or at risk of developing a neoplastic disease
       towards a given mode of treatment

<130>  SD40183

<160>  81

<170>  PatentIn version 3.3

<210>  1
<211>  28
<212>  DNA
<213>  Artificial

<220>
<223>  VEGFA

<400>  1
cacattgttg gaagaagcag cccatgac                                          28


<210>  2
<211>  18
<212>  DNA
<213>  artificial

<220>
<223>  VEGFA

<400>  2
cagatgtccc ggcgaaga                                                     18


<210>  3
<211>  18
<212>  DNA
<213>  artificial

<220>
<223>  VEGFA

<400>  3
gagggcgagt cccaggaa                                                     18


<210>  4
<211>  25
<212>  DNA
<213>  artificial

<220>
<223>  VEGFA

<400>  4
cgttcgttta actcaagctg cctcg                                             25


<210>  5
<211>  20
<212>  DNA
<213>  artificial

<220>

<223>   VEGFA

<400>   5
aacacagact cgcgttgcaa                                                    20


<210>   6
<211>   21
<212>   DNA
<213>   artificial

<220>
<223>   VEGFA

<400>   6
cggcttgtca catctgcaag t                                                  21


<210>   7
<211>   31
<212>   DNA
<213>   artificial

<220>
<223>   VEGFA

<400>   7
aacttcctcg ggttcataac catagcagtc c                                       31


<210>   8
<211>   22
<212>   DNA
<213>   artificial

<220>
<223>   VEGFA

<400>   8
cccccaacat ctggttagtc tt                                                 22


<210>   9
<211>   19
<212>   DNA
<213>   artificial

<220>
<223>   VEGFA

<400>   9
ccacgggcac agaatatgc                                                     19


<210>   10
<211>   28
<212>   DNA
<213>   artificial

<220>
<223>   VEGFA

<400>   10
caccatgcag attatgcgga tcaaacct                                           28


<210>   11
<211>   20

<212>  DNA
<213>  artificial

<220>
<223>  VEGFA

<400>  11
gcccactgag gagtccaaca                                                    20


<210>  12
<211>  20
<212>  DNA
<213>  artificial

<220>
<223>  VEGFA

<400>  12
tcctatgtgc tggccttggt                                                    20


<210>  13
<211>  28
<212>  DNA
<213>  artificial

<220>
<223>  VEGFA

<400>  13
caccatgcag attatgcgga tcaaacct                                           28


<210>  14
<211>  20
<212>  DNA
<213>  artificial

<220>
<223>  VEGFA

<400>  14
gcccactgag gagtccaaca                                                    20


<210>  15
<211>  20
<212>  DNA
<213>  artificial

<220>
<223>  VEGFA

<400>  15
gcctcggctt gtcacatttt                                                    20


<210>  16
<211>  28
<212>  DNA
<213>  artificial

<220>
<223>  VEGFA

<400>  16
caccatgcag attatgcgga tcaaacct                                           28

<210>  17
<211>  20
<212>  DNA
<213>  artificial

<220>
<223>  VEGFA

<400>  17
gcccactgag gagtccaaca                                                    20


<210>  18
<211>  20
<212>  DNA
<213>  artificial

<220>
<223>  VEGFA

<400>  18
agcaaggccc acagggattt                                                    20


<210>  19
<211>  28
<212>  DNA
<213>  artificial

<220>
<223>  VEGFA

<400>  19
caccatgcag attatgcgga tcaaacct                                           28


<210>  20
<211>  20
<212>  DNA
<213>  artificial

<220>
<223>  VEGFA

<400>  20
gcccactgag gagtccaaca                                                    20


<210>  21
<211>  21
<212>  DNA
<213>  artificial

<220>
<223>  VEGFA

<400>  21
aacgctccag gacttatacc g                                                  21


<210>  22
<211>  21
<212>  DNA
<213>  artificial

<220>

<223>  VEGFB

<400>  22
acagggctgc cactccccac c                                              21


<210>  23
<211>  26
<212>  DNA
<213>  artificial

<220>
<223>  VEGFB

<400>  23
aatgcagacc taaaaaaaag gacagt                                         26


<210>  24
<211>  17
<212>  DNA
<213>  artificial

<220>
<223>  VEGFB

<400>  24
cccagcccgg aacagaa                                                   17


<210>  25
<211>  31
<212>  DNA
<213>  artificial

<220>
<223>  VEGFB

<400>  25
cacatctatc catgacacca ctttcctctg g                                   31


<210>  26
<211>  19
<212>  DNA
<213>  artificial

<220>
<223>  VEGFB

<400>  26
tggcaggtag cgcgagtat                                                 19


<210>  27
<211>  19
<212>  DNA
<213>  artificial

<220>
<223>  VEGFB

<400>  27
ccctgtctcc cagcctgat                                                 19


<210>  28
<211>  30

<212>  DNA
<213>  artificial

<220>
<223>  VEGFB

<400>  28
ttcctcccct cactaagaag acccaaacct                                        30


<210>  29
<211>  24
<212>  DNA
<213>  artificial

<220>
<223>  VEGFB

<400>  29
ccactctgtg caagtaagca tctt                                              24


<210>  30
<211>  22
<212>  DNA
<213>  artificial

<220>
<223>  VEGFB

<400>  30
gtaccaaagc ccaaatccca tt                                                22


<210>  31
<211>  39
<212>  DNA
<213>  artificial

<220>
<223>  VEGFD

<400>  31
tgacattgaa acactaaaag ttatagatga agaatggca                              39


<210>  32
<211>  22
<212>  DNA
<213>  artificial

<220>
<223>  VEGFD

<400>  32
actaggtttg cggcaacttt ct                                                22


<210>  33
<211>  23
<212>  DNA
<213>  artificial

<220>
<223>  VEGFD

<400>  33
tctctagggc tgcactgagt tct                                               23

<210>  34
<211>  21
<212>  DNA
<213>  artificial

<220>
<223>  VEGFC

<400>  34
acggccatgt acgaaccgcc a                                                21


<210>  35
<211>  20
<212>  DNA
<213>  artificial

<220>
<223>  VEGFC

<400>  35
gttccaccac caaacatgca                                                  20


<210>  36
<211>  25
<212>  DNA
<213>  artificial

<220>
<223>  VEGFC

<400>  36
cactatatga aaatcctggc tcaca                                            25


<210>  37
<211>  21
<212>  DNA
<213>  artificial

<220>
<223>  VEGFC

<400>  37
aaacatggcc cggcgtcaac c                                                21


<210>  38
<211>  25
<212>  DNA
<213>  artificial

<220>
<223>  VEGFC

<400>  38
ccagaataga agtcatgctt tgatg                                            25


<210>  39
<211>  25
<212>  DNA
<213>  artificial

<220>

<223>  VEGFC

<400>  39
tttagatcag agcaaatgtc ttgca                                          25


<210>  40
<211>  27
<212>  DNA
<213>  artificial

<220>
<223>  VEGFC

<400>  40
ttgagtcatc tccagcatcc gaggaaa                                        27


<210>  41
<211>  22
<212>  DNA
<213>  artificial

<220>
<223>  VEGFC

<400>  41
ccacagatgt catggaatcc at                                             22


<210>  42
<211>  20
<212>  DNA
<213>  artificial

<220>
<223>  VEGFC

<400>  42
tgcctggctc aggaagattt                                                20


<210>  43
<211>  29
<212>  DNA
<213>  artificial

<220>
<223>  VEGFC

<400>  43
agaacaggcc aacctcaact caaggacag                                      29


<210>  44
<211>  21
<212>  DNA
<213>  artificial

<220>
<223>  VEGFC

<400>  44
gagatcccca tggaggtctt c                                              21


<210>  45
<211>  32

<212>  DNA
<213>  artificial

<220>
<223>  VEGFC

<400>  45
cactcattat caatactttt caagatctct gt                                    32


<210>  46
<211>  26
<212>  DNA
<213>  artificial

<220>
<223>  VEGFC

<400>  46
tgcatgccac gggaggtgtg tataga                                           26


<210>  47
<211>  24
<212>  DNA
<213>  artificial

<220>
<223>  VEGFC

<400>  47
aatagaccct ggagtgaaac catt                                             24


<210>  48
<211>  20
<212>  DNA
<213>  artificial

<220>
<223>  VEGFC

<400>  48
tattgcagca acccccacat                                                  20


<210>  49
<211>  27
<212>  DNA
<213>  artificial

<220>
<223>  VEGFC

<400>  49
acatgcagct gttacagacg gccatgt                                          27


<210>  50
<211>  21
<212>  DNA
<213>  artificial

<220>
<223>  VEGFC

<400>  50
ctgagcaagc ggtctctgag t                                                21

<210>  51
<211>  25
<212>  DNA
<213>  artificial

<220>
<223>  VEGFC

<400>  51
cactatatga aaatcctggc tcaca                                          25


<210>  52
<211>  37
<212>  DNA
<213>  artificial

<220>
<223>  VEGF-D

<400>  52
ccatcctcta ccagaacata catcagttat ttggaga                             37


<210>  53
<211>  20
<212>  DNA
<213>  artificial

<220>
<223>  VEGF-D

<400>  53
cccttcccac caagtgttca                                                20


<210>  54
<211>  19
<212>  DNA
<213>  artificial

<220>
<223>  VEGF-D

<400>  54
tggtgctgcc tcactggat                                                 19


<210>  55
<211>  25
<212>  DNA
<213>  artificial

<220>
<223>  FLT-1

<400>  55
ccaggccatg atggccttac actga                                          25


<210>  56
<211>  27
<212>  DNA
<213>  artificial

<220>

<223>  FLT-1

<400>  56
ctgaaaactt agccagagtt aggttgt                                          27


<210>  57
<211>  33
<212>  DNA
<213>  artificial

<220>
<223>  FLT-1

<400>  57
cagaataata ccaagaaatt gagttaagtg tct                                   33


<210>  58
<211>  25
<212>  DNA
<213>  artificial

<220>
<223>  FLT-1

<400>  58
atcccaagaa acctccccag acctt                                            25


<210>  59
<211>  22
<212>  DNA
<213>  artificial

<220>
<223>  FLT-1

<400>  59
tgcatcacgt agggtgactt ct                                               22


<210>  60
<211>  21
<212>  DNA
<213>  artificial

<220>
<223>  FLT-1

<400>  60
gagccagact tctcccatgg t                                                21


<210>  61
<211>  27
<212>  DNA
<213>  artificial

<220>
<223>  FLT-1

<400>  61
tgctgtcgcc ctggtagtca tcaaaca                                          27


<210>  62
<211>  19

<212>  DNA
<213>  artificial

<220>
<223>  FLT-1

<400>  62
catgggagag gccaacaga                                                    19


<210>  63
<211>  26
<212>  DNA
<213>  artificial

<220>
<223>  FLT-1

<400>  63
aacctttgaa gaacttttac cgaatg                                            26


<210>  64
<211>  27
<212>  DNA
<213>  artificial

<220>
<223>  KDR, FLK1

<400>  64
ttcttggcat cgcgaaagtg tatccac                                           27


<210>  65
<211>  24
<212>  DNA
<213>  artificial

<220>
<223>  KDR, FLK1

<400>  65
tgttacagct tccaagtggc taag                                              24


<210>  66
<211>  26
<212>  DNA
<213>  artificial

<220>
<223>  KDR, FLK1

<400>  66
attttaacca cgttcttctc cgataa                                            26


<210>  67
<211>  23
<212>  DNA
<213>  artificial

<220>
<223>  FLT4

<400>  67
cagtggcaat acggaggcaa ccg                                               23

<210>  68
<211>  28
<212>  DNA
<213>  artificial

<220>
<223>  FLT4

<400>  68
ttaacctctg tgtgctagct ttctatct                                          28


<210>  69
<211>  20
<212>  DNA
<213>  artificial

<220>
<223>  FLT4

<400>  69
cctacgcatg tgtgcattcc                                                   20


<210>  70
<211>  23
<212>  DNA
<213>  artificial

<220>
<223>  FLT4

<400>  70
tgcctgcttc cctgggtagt ccc                                               23


<210>  71
<211>  17
<212>  DNA
<213>  artificial

<220>
<223>  FLT4

<400>  71
gcacccactt accccgc                                                      17


<210>  72
<211>  26
<212>  DNA
<213>  artificial

<220>
<223>  FLT4

<400>  72
gagtttaact caggtgtcac ctttga                                            26


<210>  73
<211>  28
<212>  DNA
<213>  artificial

<220>

<223>  FLT4

<400>  73
aagggatttt catgcacaat gacctcgg                                          28


<210>  74
<211>  19
<212>  DNA
<213>  artificial

<220>
<223>  FLT4

<400>  74
tgagccactc gacgctgat                                                    19


<210>  75
<211>  18
<212>  DNA
<213>  artificial

<220>
<223>  FLT4

<400>  75
cagcgatttc gggagagc                                                     18


<210>  76
<211>  29
<212>  DNA
<213>  artificial

<220>
<223>  FLT4

<400>  76
aagaaagcgg cttcagctgt aaaggacct                                         29


<210>  77
<211>  24
<212>  DNA
<213>  artificial

<220>
<223>  FLT4

<400>  77
tgagcagata gagagcaggc atag                                              24


<210>  78
<211>  20
<212>  DNA
<213>  artificial

<220>
<223>  FLT4

<400>  78
ccctggtcac agccacattc                                                   20


<210>  79
<211>  29

<212>  DNA
<213>  artificial

<220>
<223>  FLT4

<400>  79
aggcatagac aagaaagcgg cttcaggta                                          29


<210>  80
<211>  23
<212>  DNA
<213>  artificial

<220>
<223>  FLT4

<400>  80
ctcggaggag tttgagcaga tag                                                23


<210>  81
<211>  23
<212>  DNA
<213>  artificial

<220>
<223>  FLT4

<400>  81
ctgacgtatg ctgccttctc tct                                                23

SEQUENCE LISTING

<110> Siemens AG

<120> A method for predicting a clinical response of a patient
      suffering from or at risk of developing a neoplastic disease
      towards a given mode of treatment

<130> SD40183

<160> 81

<170> PatentIn version 3.3

<210> 1
<211> 28
<212> DNA
<213> Artificial

<220>
<223> VEGFA

<400> 1
cacattgttg gaagaagcag cccatgac                                    28


<210> 2
<211> 18
<212> DNA
<213> artificial

<220>
<223> VEGFA

<400> 2
cagatgtccc ggcgaaga                                               18


<210> 3
<211> 18
<212> DNA
<213> artificial

<220>
<223> VEGFA

<400> 3
gagggcgagt cccaggaa                                               18


<210> 4
<211> 25
<212> DNA
<213> artificial

<220>
<223> VEGFA

<400> 4
cgttcgttta actcaagctg cctcg                                       25


<210> 5
<211> 20
<212> DNA
<213> artificial

<220>

<223>  VEGFA

<400>  5
aacacagact cgcgttgcaa                                                    20


<210>  6
<211>  21
<212>  DNA
<213>  artificial

<220>
<223>  VEGFA

<400>  6
cggcttgtca catctgcaag t                                                  21


<210>  7
<211>  31
<212>  DNA
<213>  artificial

<220>
<223>  VEGFA

<400>  7
aacttcctcg ggttcataac catagcagtc c                                       31


<210>  8
<211>  22
<212>  DNA
<213>  artificial

<220>
<223>  VEGFA

<400>  8
cccccaacat ctggttagtc tt                                                 22


<210>  9
<211>  19
<212>  DNA
<213>  artificial

<220>
<223>  VEGFA

<400>  9
ccacgggcac agaatatgc                                                     19


<210>  10
<211>  28
<212>  DNA
<213>  artificial

<220>
<223>  VEGFA

<400>  10
caccatgcag attatgcgga tcaaacct                                           28


<210>  11
<211>  20

<212>  DNA
<213>  artificial

<220>
<223>  VEGFA

<400>  11
gcccactgag gagtccaaca                                                    20


<210>  12
<211>  20
<212>  DNA
<213>  artificial

<220>
<223>  VEGFA

<400>  12
tcctatgtgc tggccttggt                                                    20


<210>  13
<211>  28
<212>  DNA
<213>  artificial

<220>
<223>  VEGFA

<400>  13
caccatgcag attatgcgga tcaaacct                                           28


<210>  14
<211>  20
<212>  DNA
<213>  artificial

<220>
<223>  VEGFA

<400>  14
gcccactgag gagtccaaca                                                    20


<210>  15
<211>  20
<212>  DNA
<213>  artificial

<220>
<223>  VEGFA

<400>  15
gcctcggctt gtcacatttt                                                    20


<210>  16
<211>  28
<212>  DNA
<213>  artificial

<220>
<223>  VEGFA

<400>  16
caccatgcag attatgcgga tcaaacct                                           28

<210> 17
<211> 20
<212> DNA
<213> artificial

<220>
<223> VEGFA

<400> 17
gcccactgag gagtccaaca 20

<210> 18
<211> 20
<212> DNA
<213> artificial

<220>
<223> VEGFA

<400> 18
agcaaggccc acagggattt 20

<210> 19
<211> 28
<212> DNA
<213> artificial

<220>
<223> VEGFA

<400> 19
caccatgcag attatgcgga tcaaacct 28

<210> 20
<211> 20
<212> DNA
<213> artificial

<220>
<223> VEGFA

<400> 20
gcccactgag gagtccaaca 20

<210> 21
<211> 21
<212> DNA
<213> artificial

<220>
<223> VEGFA

<400> 21
aacgctccag gacttatacc g 21

<210> 22
<211> 21
<212> DNA
<213> artificial

<220>

<223>  VEGFB

<400>  22
acagggctgc cactccccac c                                                21


<210>  23
<211>  26
<212>  DNA
<213>  artificial

<220>
<223>  VEGFB

<400>  23
aatgcagacc taaaaaaaag gacagt                                           26


<210>  24
<211>  17
<212>  DNA
<213>  artificial

<220>
<223>  VEGFB

<400>  24
cccagcccgg aacagaa                                                     17


<210>  25
<211>  31
<212>  DNA
<213>  artificial

<220>
<223>  VEGFB

<400>  25
cacatctatc catgacacca ctttcctctg g                                     31


<210>  26
<211>  19
<212>  DNA
<213>  artificial

<220>
<223>  VEGFB

<400>  26
tggcaggtag cgcgagtat                                                   19


<210>  27
<211>  19
<212>  DNA
<213>  artificial

<220>
<223>  VEGFB

<400>  27
ccctgtctcc cagcctgat                                                   19


<210>  28
<211>  30

<212>  DNA
<213>  artificial

<220>
<223>  VEGFB

<400>  28
ttcctcccct cactaagaag acccaaacct                                        30


<210>  29
<211>  24
<212>  DNA
<213>  artificial

<220>
<223>  VEGFB

<400>  29
ccactctgtg caagtaagca tctt                                              24


<210>  30
<211>  22
<212>  DNA
<213>  artificial

<220>
<223>  VEGFB

<400>  30
gtaccaaagc ccaaatccca tt                                                22


<210>  31
<211>  39
<212>  DNA
<213>  artificial

<220>
<223>  VEGFD

<400>  31
tgacattgaa acactaaaag ttatagatga agaatggca                              39


<210>  32
<211>  22
<212>  DNA
<213>  artificial

<220>
<223>  VEGFD

<400>  32
actaggtttg cggcaacttt ct                                                22


<210>  33
<211>  23
<212>  DNA
<213>  artificial

<220>
<223>  VEGFD

<400>  33
tctctagggc tgcactgagt tct                                               23

<210> 34
<211> 21
<212> DNA
<213> artificial

<220>
<223> VEGFC

<400> 34
acggccatgt acgaaccgcc a 21


<210> 35
<211> 20
<212> DNA
<213> artificial

<220>
<223> VEGFC

<400> 35
gttccaccac caaacatgca 20


<210> 36
<211> 25
<212> DNA
<213> artificial

<220>
<223> VEGFC

<400> 36
cactatatga aaatcctggc tcaca 25


<210> 37
<211> 21
<212> DNA
<213> artificial

<220>
<223> VEGFC

<400> 37
aaacatggcc cggcgtcaac c 21


<210> 38
<211> 25
<212> DNA
<213> artificial

<220>
<223> VEGFC

<400> 38
ccagaataga agtcatgctt tgatg 25


<210> 39
<211> 25
<212> DNA
<213> artificial

<220>

<223>  VEGFC

<400>  39
tttagatcag agcaaatgtc ttgca                                          25


<210>  40
<211>  27
<212>  DNA
<213>  artificial

<220>
<223>  VEGFC

<400>  40
ttgagtcatc tccagcatcc gaggaaa                                        27


<210>  41
<211>  22
<212>  DNA
<213>  artificial

<220>
<223>  VEGFC

<400>  41
ccacagatgt catggaatcc at                                             22


<210>  42
<211>  20
<212>  DNA
<213>  artificial

<220>
<223>  VEGFC

<400>  42
tgcctggctc aggaagattt                                                20


<210>  43
<211>  29
<212>  DNA
<213>  artificial

<220>
<223>  VEGFC

<400>  43
agaacaggcc aacctcaact caaggacag                                      29


<210>  44
<211>  21
<212>  DNA
<213>  artificial

<220>
<223>  VEGFC

<400>  44
gagatcccca tggaggtctt c                                              21


<210>  45
<211>  32

<212>  DNA
<213>  artificial

<220>
<223>  VEGFC

<400>  45
cactcattat caatactttt caagatctct gt                                    32


<210>  46
<211>  26
<212>  DNA
<213>  artificial

<220>
<223>  VEGFC

<400>  46
tgcatgccac gggaggtgtg tataga                                           26


<210>  47
<211>  24
<212>  DNA
<213>  artificial

<220>
<223>  VEGFC

<400>  47
aatagaccct ggagtgaaac catt                                             24


<210>  48
<211>  20
<212>  DNA
<213>  artificial

<220>
<223>  VEGFC

<400>  48
tattgcagca acccccacat                                                  20


<210>  49
<211>  27
<212>  DNA
<213>  artificial

<220>
<223>  VEGFC

<400>  49
acatgcagct gttacagacg gccatgt                                          27


<210>  50
<211>  21
<212>  DNA
<213>  artificial

<220>
<223>  VEGFC

<400>  50
ctgagcaagc ggtctctgag t                                                21

<210> 51
<211> 25
<212> DNA
<213> artificial

<220>
<223> VEGFC

<400> 51
cactatatga aaatcctggc tcaca 25

<210> 52
<211> 37
<212> DNA
<213> artificial

<220>
<223> VEGF-D

<400> 52
ccatcctcta ccagaacata catcagttat ttggaga 37

<210> 53
<211> 20
<212> DNA
<213> artificial

<220>
<223> VEGF-D

<400> 53
cccttcccac caagtgttca 20

<210> 54
<211> 19
<212> DNA
<213> artificial

<220>
<223> VEGF-D

<400> 54
tggtgctgcc tcactggat 19

<210> 55
<211> 25
<212> DNA
<213> artificial

<220>
<223> FLT-1

<400> 55
ccaggccatg atggccttac actga 25

<210> 56
<211> 27
<212> DNA
<213> artificial

<220>

<223>  FLT-1

<400>  56
ctgaaaactt agccagagtt aggttgt                                          27


<210>  57
<211>  33
<212>  DNA
<213>  artificial

<220>
<223>  FLT-1

<400>  57
cagaataata ccaagaaatt gagttaagtg tct                                   33


<210>  58
<211>  25
<212>  DNA
<213>  artificial

<220>
<223>  FLT-1

<400>  58
atcccaagaa acctccccag acctt                                            25


<210>  59
<211>  22
<212>  DNA
<213>  artificial

<220>
<223>  FLT-1

<400>  59
tgcatcacgt agggtgactt ct                                               22


<210>  60
<211>  21
<212>  DNA
<213>  artificial

<220>
<223>  FLT-1

<400>  60
gagccagact tctcccatgg t                                                21


<210>  61
<211>  27
<212>  DNA
<213>  artificial

<220>
<223>  FLT-1

<400>  61
tgctgtcgcc ctggtagtca tcaaaca                                          27


<210>  62
<211>  19

<212>  DNA
<213>  artificial

<220>
<223>  FLT-1

<400>  62
catgggagag gccaacaga                                                    19


<210>  63
<211>  26
<212>  DNA
<213>  artificial

<220>
<223>  FLT-1

<400>  63
aacctttgaa gaacttttac cgaatg                                            26


<210>  64
<211>  27
<212>  DNA
<213>  artificial

<220>
<223>  KDR, FLK1

<400>  64
ttcttggcat cgcgaaagtg tatccac                                           27


<210>  65
<211>  24
<212>  DNA
<213>  artificial

<220>
<223>  KDR, FLK1

<400>  65
tgttacagct tccaagtggc taag                                              24


<210>  66
<211>  26
<212>  DNA
<213>  artificial

<220>
<223>  KDR, FLK1

<400>  66
attttaacca cgttcttctc cgataa                                            26


<210>  67
<211>  23
<212>  DNA
<213>  artificial

<220>
<223>  FLT4

<400>  67
cagtggcaat acggaggcaa ccg                                               23

<210> 68
<211> 28
<212> DNA
<213> artificial

<220>
<223> FLT4

<400> 68
ttaacctctg tgtgctagct ttctatct 28

<210> 69
<211> 20
<212> DNA
<213> artificial

<220>
<223> FLT4

<400> 69
cctacgcatg tgtgcattcc 20

<210> 70
<211> 23
<212> DNA
<213> artificial

<220>
<223> FLT4

<400> 70
tgcctgcttc cctgggtagt ccc 23

<210> 71
<211> 17
<212> DNA
<213> artificial

<220>
<223> FLT4

<400> 71
gcacccactt accccgc 17

<210> 72
<211> 26
<212> DNA
<213> artificial

<220>
<223> FLT4

<400> 72
gagtttaact caggtgtcac ctttga 26

<210> 73
<211> 28
<212> DNA
<213> artificial

<220>

<223>  FLT4

<400>  73
aagggatttt catgcacaat gacctcgg                                          28


<210>  74
<211>  19
<212>  DNA
<213>  artificial

<220>
<223>  FLT4

<400>  74
tgagccactc gacgctgat                                                    19


<210>  75
<211>  18
<212>  DNA
<213>  artificial

<220>
<223>  FLT4

<400>  75
cagcgatttc gggagagc                                                     18


<210>  76
<211>  29
<212>  DNA
<213>  artificial

<220>
<223>  FLT4

<400>  76
aagaaagcgg cttcagctgt aaaggacct                                         29


<210>  77
<211>  24
<212>  DNA
<213>  artificial

<220>
<223>  FLT4

<400>  77
tgagcagata gagagcaggc atag                                              24


<210>  78
<211>  20
<212>  DNA
<213>  artificial

<220>
<223>  FLT4

<400>  78
ccctggtcac agccacattc                                                   20


<210>  79
<211>  29

<212>  DNA
<213>  artificial

<220>
<223>  FLT4

<400>  79
aggcatagac aagaaagcgg cttcaggta                                    29


<210>  80
<211>  23
<212>  DNA
<213>  artificial

<220>
<223>  FLT4

<400>  80
ctcggaggag tttgagcaga tag                                          23


<210>  81
<211>  23
<212>  DNA
<213>  artificial

<220>
<223>  FLT4

<400>  81
ctgacgtatg ctgccttctc tct                                          23